# EUROPEAN PATENT SPECIFICATION

(11) **EP 4 282 947 B1**
(45) Date of publication and mention of the grant of the patent: **02.09.2026**
(21) Application number: 21920969.9
(22) Date of filing: 20.01.2021
(51) Int. Cl.: C12M 3/00, G06T 7/00

(54) **EMBRYO CULTURING APPARATUS AND METHOD FOR DISPLAYING TREATED EGG MAINTAINED IN CULTURE ENVIRONMENT**
EMBRYONENKULTIVIERUNGSVORRICHTUNG UND VERFAHREN ZUR ANZEIGE VON BEHANDELTEM EI, DAS IN DER KULTURUMGEBUNG GEHALTEN WIRD
APPAREIL DE CULTURE D'EMBRYONS ET PROCÉDÉ D'AFFICHAGE D'OEUFS TRAITÉS MAINTENUS DANS UN ENVIRONNEMENT DE CULTURE

(43) Date of publication of application: 29.11.2023
(73) Proprietor: Asada Ladies Clinic, Nagoya-shi, Aichi, 450-0002 (JP)
(72) Inventor: FUKUNAGA Noritaka, Nagoya-shi, Aichi 450-0002 (JP)
(74) Representative: Kuhnen & Wacker Patent- und Rechtsanwaltsbüro PartG mbB
(86) International application number: PCT/JP2021/001849
(87) International publication number: WO 2022/157855

(56) References cited:
- WO-A1-2011/004568
- WO-A1-2019/225176
- AU-A1- 2019 273 148
- JP-A- 2018 093 795
- JP-A- 2018 093 795
- JP-A- 2020 054 247

## Description

### BACKGROUND

### Field

The present disclosure relates to a technique that cultures an embryo.

### Related Art

There is an increasing social demand for infertility care, and various devices that culture inseminated ova (hereinafter simply referred treatment eggs) under microscopes or the like are proposed or sold. In recent years, devices that each include a camera provided in such a culture device to image a treatment egg during cultured, and to display a captured image on an external display as needed are also becoming popular (see, for example, JP 2012-39929 A). In such an embryo culture device, a treatment egg is placed in a tray (also referred to as a dish) that stably holds the treatment egg, is transported into a visual field of the camera at a predetermined interval together with the tray, and is imaged. An embryologist can decide whether or not fertilization succeeds, by observing this image, and consequently does not need to take the treatment egg outside of the culture device only to observe. An embryo culture device according to the preamble of claim 1 is disclosed by JP 2018-093795 A.

On the other hand, in response to increase in fertility treatment, there is a demand that one embryo culture device cultures multiple treatment eggs, and a plurality of trays are accommodated in the embryo culture device. In such a case, the following problems occur when treatment eggs subjected to insemination treatment are cultured.
[1] A state of a treatment egg that is being cultured differs per treatment egg. Times taken until fertilization, and a progress of subsequent cleavage are different even between treatment eggs that are subjected to insemination processing at the same period. Hence, when the number of treatment eggs that are being cultured increases, work of accurately determining this situation, and providing the treatment eggs for cryopreservation and embryo transfer of fertilized eggs becomes very complicated. Hence, a person (mainly an embryologist) who cultures a treatment egg has had to determine a situation of a treatment egg in a short time.
[2] In a case where a situation of a treatment egg is determined looking at an image obtained by imaging the treatment egg, if there are multiple images, it is not possible to easily determine which image to use to determine the situation of the treatment egg. An imaging time interval may be shorter to reliably grasp the situation of the treatment egg, and it is also desirable to continuously image the situation as a so-called movie. However, when the imaging time interval becomes short, the number of images that need to be checked increases. As a result, there is a concern that accuracy of determination made looking at images lowers, a time is taken to make a determination, and determination on other treatment eggs cannot be made or is delayed.

### SUMMARY

The present disclosure has been made to solve at least part of the above-described problem and can achieve the following aspects and application examples.
(1) Various aspects of the present disclosure will be described below. The first aspect is an aspect of an embryo culture device that keeps in a culture environment a treatment egg having been subjected to insemination treatment. This embryo culture device includes: a culture unit that accommodates a plurality of recess parts that can each accommodate a treatment egg, and keeps the recess parts in the culture environment; an imaging unit that continuously or intermittently captures an image including the treatment egg accommodated in the recess part without relatively moving the recess part; a storage unit that stores per treatment egg an egg image that is an image including the imaged treatment egg; a determination unit that applies a learned model generated in advance by supervised machine learning to the egg image of each treatment egg, and makes a determination of growth of the treatment egg; and a display unit that reads from the storage unit an egg image of a treatment egg having been determined to satisfy a condition set in advance according to a result of the determination to display. The display unit includes an acceptance unit that displays the result of the determination and the egg image used for the determination, and then accepts an applicability decision of an embryologist on the result of the determination. The embryo culture device further comprises a relearning unit that, when the applicability decision of the embryologist accepted by the acceptance unit denies the result of the determination displayed on the display unit, relearns and updates the learned model. This embryo culture device can make a determination of growth of treatment eggs using images obtained by imaging the treatment eggs accommodated and cultured in the recess parts without moving the treatment eggs relatively with respect to the imaging units, so that it is possible to reduce fluctuation of environment that is disadvantageous for the treatment eggs that are being cultured, and, moreover, accurately grasp situations of treatment eggs that are being cultured and take necessary measures.
(2) The second aspect is an aspect of a method for displaying a treatment egg kept in a culture environment and having been subjected to insemination treatment. This method includes: accommodating a plurality of recess parts that can each accommodate a treatment egg, and keeping the recess parts in the culture environment; preparing an imaging unit at a predetermined position with respect to the recess part; continuously or intermittently capturing an image including the treatment egg accommodated in the recess part using the imaging unit; storing in a storage unit per treatment egg an egg image that is an image including the imaged treatment egg; applying a learned model generated in advance by supervised machine learning to the egg image of the treatment egg, and making a determination of growth of the treatment egg; and reading from the storage unit an egg image of a treatment egg having been determined to satisfy a condition set in advance according to a result of the determination to display on a display unit. The method further comprising displaying the result of the determination and the egg image used for the determination, and then accepting an applicability decision of an embryologist on the result of the determination, and when the applicability decision of the embryologist accepted by the acceptance unit denies the result of the determination displayed on the display unit, relearning and updating the learned model. By so doing, it is possible to display an image based on which determination of growth of treatment eggs has been made using images obtained by the imaging units prepared at predetermined positions by imaging the treatment eggs accommodated and cultured in the recess parts, so that it is possible to reduce fluctuation of environment that is disadvantageous for the treatment eggs that are being cultured, and, moreover, accurately grasp situations of the treatment eggs that are being cultured and provide necessary display.

The present disclosure can be carried out not only as such an embryo culture device and a method for displaying a treatment egg placed in a culture environment, but also as other aspects. The present disclosure can be carried out as, for example, an embryo culture method, an imaging device of the embryo culture device, a method for manufacturing the embryo culture device, a method for observing treatment eggs accommodated in the embryo culture device, or a fertilization determination device or a method thereof for the treatment eggs.

### BRIEF DESCRIPTION OF THE DRAWINGS

FIG. 1 is a plan view of an embryo culture device according to a first embodiment;
FIG. 2 is a perspective view illustrating part of the embryo culture device;
FIG. 3 is a plan view illustrating one of culture chambers;
FIG. 4 is an explanatory view illustrating an electric configuration of the embryo culture device that includes a time lapse unit;
FIG. 5 is a plan view illustrating an example of a tray that accommodates a treatment egg;
FIG. 6 is an explanatory view for describing a relationship between the time lapse unit and a well of the tray;
FIG. 7A is an explanatory view illustrating an internal configuration of the time lapse unit;
FIG. 7B is an explanatory view illustrating a configuration of a camera module;
FIG. 8 is a flowchart illustrating a culture unit control routine;
FIG. 9 is an explanatory view illustrating display examples at a normal time and an abnormal time of a display module;
FIG. 10 is a flowchart illustrating a learned model generation processing routine;
FIG. 11A is an explanatory view illustrating an image labeled as an egg from which the pronucleus has not been confirmed;
FIG. 11B is an explanatory view illustrating an image labeled as an egg from which one pronucleus has been confirmed;
FIG. 11C is an explanatory view illustrating an image labeled as an egg from which two pronuclei have been confirmed;
FIG. 11D is an explanatory view illustrating an image labeled as an egg from three pronuclei have been confirmed;
FIG. 12A is an explanatory view illustrating an image labeled as an embryo in a state where cleavage after insemination processing is not started;
FIG. 12B is an explanatory view illustrating an image labeled as an embryo that has directly transitioned to a state where the number of blastomeres is two during cleavage after insemination processing;
FIG. 12C is an explanatory view illustrating an image labeled as an embryo that has transitioned to a state where the number of blastomeres is three during cleavage after insemination processing;
FIG. 12D is an explanatory view illustrating an image labeled as an embryo that has transitioned to a chaotic state during cleavage after insemination processing;
FIG. 13 is an explanatory view illustrating how a learned model that uses a neural network is generated by machine learning;
FIG. 14A is a flowchart illustrating a time lapse control processing routine;
FIG. 14B is a flowchart illustrating an example of a determination processing routine of the time lapse control processing;
FIG. 15 is an explanatory view illustrating an example of imaging and correction of a treatment egg in the well;
FIG. 16 is a schematic view illustrating a principle of adjusting a focus position in a case where a camera unit captures an image;
FIG. 17 is an explanatory view of a dish including a microwell that accommodates a treatment egg;
FIG. 18 is a schematic view illustrating a configuration example in a case where the camera unit is provided on a side surface of the culture chamber; and
FIG. 19 is an explanatory view illustrating a relationship between the well and the treatment egg accommodated in the well when the tray is seen from the side surface.

### DETAILED DESCRIPTION

### A. First Embodiment:

### A1. Hardware Configuration:

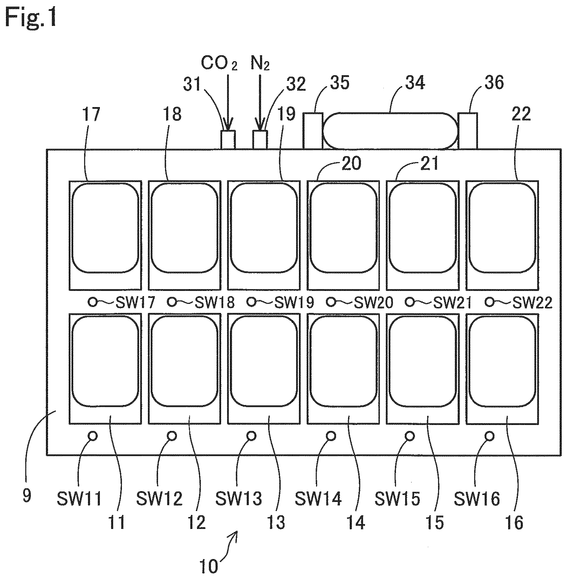

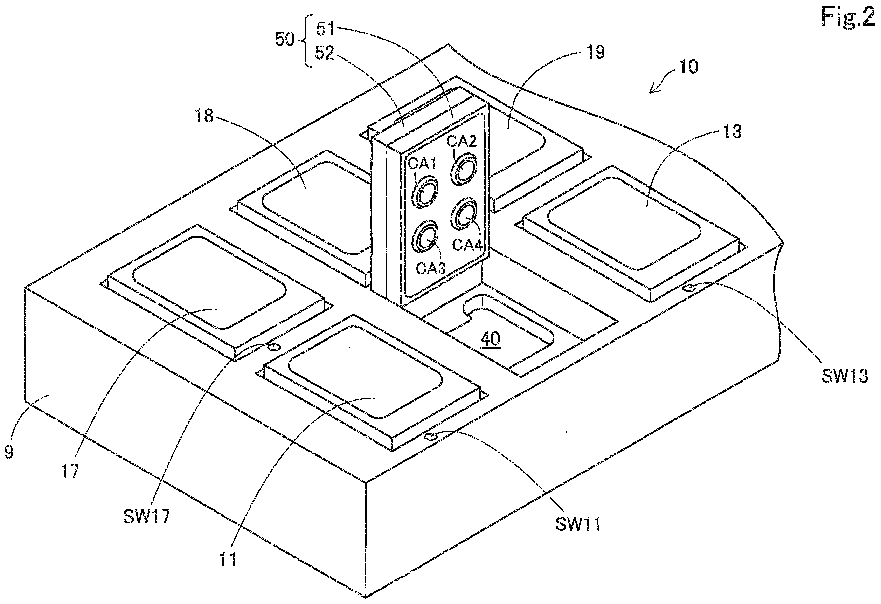

The first embodiment will be described. FIG. 1 is a plan view of an embryo culture device (also referred to as an incubator) 10, and FIG. 2 is a perspective view illustrating part of the embryo culture device 10. This embryo culture device 10 cultures an egg (hereinafter, referred to as a treatment egg) subjected to in-vitro fertilization treatment for a certain period in a predetermined culture environment, that is, at a constant temperature and a constant humidity. The treatment egg is not necessarily a fertilized egg, and therefore is cultured for a predetermined period by the embryo culture device 10. Note that there may be a case where in-vitro fertilization treatment is intracytoplasmic sperm injection performed under a microscope, or a case where in-vitro fertilization treatment is general ex-vivo fertilization performed by placing an egg and a sperm together in a predetermined container. A method for fertilizing a treatment egg to be cultured does not matter.

As illustrated in FIG. 1, this embryo culture device 10 includes 12 culture units 11 to 22. The number of culture units does not matter. A configuration and a function of each of the culture units 11 to 22 will be described later. Two front and rear rows of every six of these culture units 11 to 22 are provided in a width direction in a flat case main body 9. The respective culture units 11 to 22 are provided in association with switches SW11 to SW22, respectively. By operating these switches SW11 to SW22, a time lapse unit 50 of each of the corresponding culture units 11 to 22 is opened or closed by an unillustrated drive mechanism. The culture units 11 to 22 may also adopt a structure that is manually opened and closed.

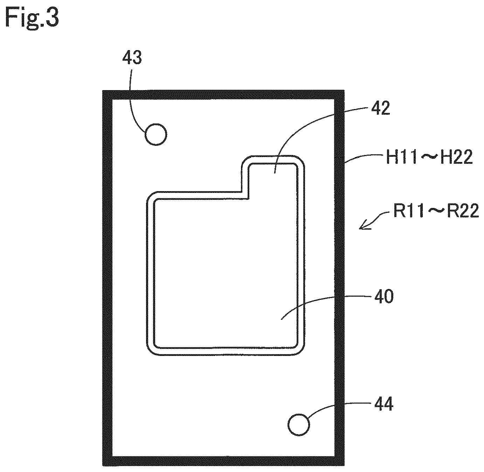

FIG. 2 illustrates that the time lapse unit 50 is in an opened state in the culture unit 12. The time lapse unit 50 includes a camera module 51 and a display module 52. The camera module 51 and the display module 52 have the same dimension, and are integrally formed. The camera module 51 is provided with four camera units CA1 to CA4. Structures and functions of these camera units CA1 to CA4 will be described later. The respective culture units 11 to 22 include culture chambers R11 to R22 (see FIG. 3) prepared therein, respectively, and the time lapse units 50 of the respective culture chambers R11 to R22 also function as lids for the corresponding culture chambers R11 to R22. An outer circumference on the camera module 51 side of the time lapse unit 50 is provided with a seal part of a silicone rubber, and, when the time lapse unit 50 is closed, the culture chambers R11 to R22 are kept in an airtight state at a predetermined atmospheric pressure or less by this seal part.

The embryo culture device 10 receives a supply of a carbon dioxide gas (CO₂) and a nitrogen gas (N₂) from an external gas cylinder. Gas ports 31 and 32 to which these gases are supplied are provided on a back surface of the embryo culture device 10. Furthermore, filter ports 35 and 36 to which a filter 34 is attached are also provided on the back surface of the embryo culture device 10. This filter 34 removes a foreign material such as dust in outdoor air taken in by an unillustrated built-in pump. The embryo culture device 10 mixes the carbon dioxide gas CO₂ and the nitrogen gas N₂ input from the gas ports 31 and 32, and air input through the filter 34 at a predetermined rate to generate a gaseous mixture. The rate of each gas in the gaseous mixture is measured by an unillustrated sensor, and is kept at the same rate at all times.

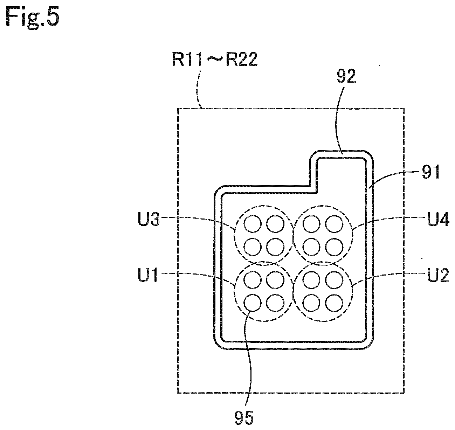

FIG. 3 is a plan view illustrating one of the culture chambers R11 to R22. As illustrated in FIG. 3, the culture chambers R11 to R22 are each provided with a dented tray accommodation part 40 in which a tray (also referred to as a dish) accommodating a treatment egg is placed. FIG. 5 illustrates a shape of a tray 91. The tray accommodation part 40 has the shape that matches the shape of the tray 91. According to the present embodiment, the tray accommodation part 40 has the shape (expanded part 42) whose one portion of the recess of a rectangular shape is expanded outward, and the tray 91 also has the shape (protrusion part 92) whose one portion protrudes outward likewise. Hence, the tray accommodation part 40 has the shape that is not symmetrical with respect to a center line on any one of front, back, left, and right sides. Hence, when the tray 91 is placed in the tray accommodation part 40, no mistake occurs regarding a direction in which the tray 91 is placed. Mistake of placement may be prevented by another method such as electrical detection, and the shapes of the tray accommodation part 40 and the tray 91 may be symmetrical with respect to the left and right sides and/or the front and rear sides. Furthermore, the tray 91 may be provided with a marker, and, arrangement (such as an orientation) of the tray 91 may be specified or the treatment egg may be directly specified from a captured image irrespectively of a placement direction.

A supply port 43 and a discharge port 44 may be provided in each of the bottom surfaces of the culture chambers R11 to R22. The supply port 43 is an opening for supplying a gaseous mixture to the culture chambers R11 to R22. Furthermore, the discharge port 44 is an opening for refluxing the gaseous mixture. Although, as described above, when the time lapse unit 50 is closed, the culture chambers R11 to R22 are placed in a substantially airtight state, and therefore a gas environment inside is kept constant, the gaseous mixture is supplied from the supply port 43 and discharged from the discharge port 44 to make temperature distributions in the culture chambers R11 to R22 uniform. Note that part of the gaseous mixture slightly leaks from a seal part sealed with the time lapse unit 50. By so doing, outdoor air is prevented from entering from the seal part sealed with the time lapse unit 50.

Each of the culture chambers R11 to R22 is provided with panel heaters H11 to H22 in a sidewall and a bottom surface of each of the culture chambers R11 to R22. The heaters H11 to H22 may be provided only in the sidewall or only in the bottom surface. Alternatively, independent heaters may be provided to the culture chambers R11 to R22. Furthermore, independent heaters may be provided on a lid side, too. The panel heaters H11 to H22 keep the interiors of the culture chambers R11 to R22 at constant temperatures. Note that, although description is omitted, the culture chambers R11 to R22 are each provided with an unillustrated temperature sensor and gas concentration sensor, and, moreover, a humidity sensor and the like, so that it is possible to detect temperatures, gas concentration levels, and humidity levels in the culture chambers R11 to R22. By feeding back signals from these sensors, constant temperatures and constant humidity levels are eventually kept in the culture chambers R11 to R22, and gas concentration levels are also kept constant. Note that, by keeping the temperature, the humidity level, the gas concentration level, and the like of the gaseous mixture supplied from the supply port 43 constant instead of detecting the temperatures and the like in the culture chambers R11 to R22, an environment in the culture chambers R11 to R22 may be kept constant.

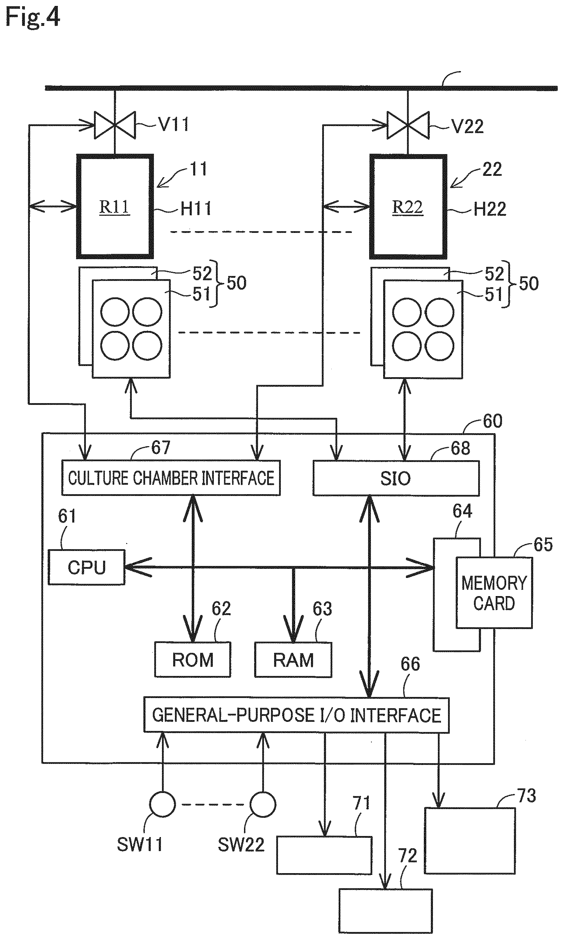

Next, the time lapse unit 50 will be described. FIG. 4 is an explanatory view illustrating an electrical configuration of the embryo culture device 10 including the time lapse units 50. As illustrated in FIG. 4, the embryo culture device 10 is provided with a control unit 60. The control unit 60 includes a known CPU 61, ROM 62, and RAM 63, and, in addition, a memory interface 64 that exchanges data with a memory card 65, a general-purpose I/O interface 66 that exchanges signals with external equipment, a culture chamber interface 67 that exchanges signals for controlling an environment in the culture chambers R11 to R22, an SIO 68 that performs serial communication with the time lapse units 50, and the like. The memory card 65 stores information related to an environment such as the temperature in each of the culture chambers R11 to R22.

The general-purpose I/O interface 66 is connected to the switches SW11 to SW22 that are provided to the embryo culture device 10, a driving device 71 that opens and closes the time lapse unit 50, a warning device 72 that issues a warning sound, a gaseous mixture adjustment device 73 that adjusts a gaseous mixture, and the like. The gaseous mixture adjustment device 73 includes a pressure adjustment valve that adjusts a pressure of a carbon dioxide gas, a nitrogen gas, or the like supplied to the gas ports 31 and 32, a pump that takes in an atmosphere through the filter 34, a pump that feeds the gaseous mixture to the culture chambers R11 to R22, and the like. The culture chamber interface 67 is connected with the panel heaters H11 to H22 that are provided to each of the culture chambers R11 to R22, control valves V11 to V22 that control supply amounts of gaseous mixtures, and the like. The control valves V11 to V22 are provided to pipes that reach the culture chambers R11 to R22 from a gaseous mixture supply pipe 84 that supplies the gaseous mixture.

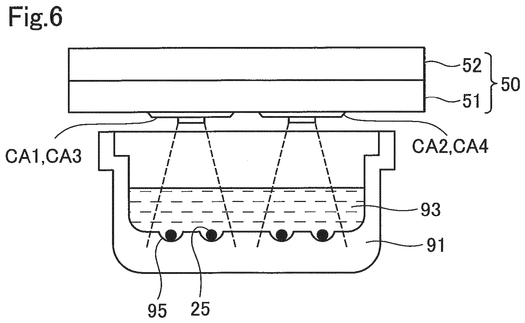

It has been already described that the camera module 51 of the time lapse unit 50 is provided with the four camera units CA1 to CA4. As illustrated in FIGS. 5 and 6, these camera units CA1 to CA4 each image wells 95 that are recess parts provided to the tray 91. Every four wells 95 are disposed collectively as a set at four portions in the tray 91 as illustrated. The camera units CA1 to CA4 are provided in association with respective sets. Areas U1 to U4 illustrated in FIG. 5 indicate respective imaging areas of the camera units CA1 to CA4. That is, each of the camera units CA1 to CA4 can image an area including the four wells 95 at once. Light guides that guide light from LEDs are provided around lenses of the camera units CA1 to CA4. The camera units CA1 to CA4 irradiate the trays 91 with light from the light guides at a time of imaging.

Each of the camera units CA1 to CA4 may image the more multiple wells 95 at once. For example, the camera unit CA1 may be provided at the center of the 16 wells 95, and the one camera unit CA1 may image the four areas U1 to U4 at once. Alternatively, only the camera unit CA1 and the camera unit CA4 may be provided, each camera unit CA1 may image the areas U1 and U2, and the camera unit CA4 may image the areas U3 and U4. Furthermore, the four camera units CA1 to CA4 may image the respectively adjacent areas U1 to U4 in an overlapping manner. By so doing, it is possible to acquire images of the wells 95 of all of the areas U1 to U4 by using a video image of the other camera unit even when one camera unit goes out of order and cannot capture an image as described below.

FIG. 6 schematically illustrates how imaging is performed. The well 95 is provided at a bottom part of the tray 91, and the one treatment egg 25 is basically placed in the one well 95, and is coated with a mineral oil 93. Each of the camera units CA1 to CA4 images the areas U1 to U4 including the four wells 95 from above the tray 91. The captured images include images of the treatment eggs 25. The camera units CA1 to CA4 can adjust focus positions at several µm in accuracy at a time of imaging. How the camera units CA1 to CA4 image the treatment eggs 25 will be described later.

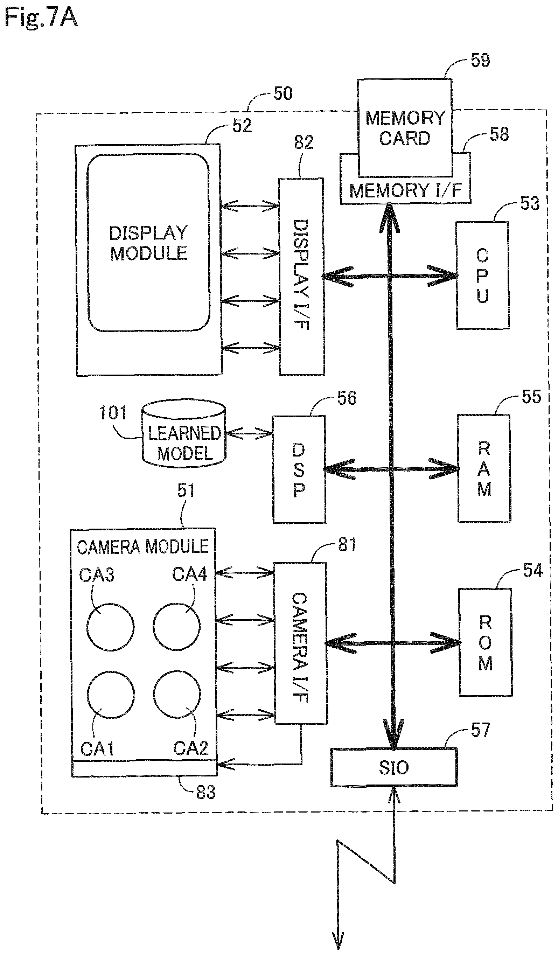

Next, an internal configuration of the time lapse unit 50 will be described. FIG. 7A is an explanatory view illustrating the internal configuration of the time lapse unit 50. As illustrated in FIG. 7A, the time lapse unit 50 includes the above-described camera module 51 and display module 52 and, in addition, a known CPU 53, ROM 54, RAM 55, a Digital Signal Processor (DSP) 56, an SIO 57, a memory interface 58, a camera interface 81, a display interface 82, and the like. The DSP 56 is a processor that can process a digital signal at a high speed, and employs a configuration that can process weighted operation processing suitable to machine learning over multiple layers. The DSP 56 can process image determination that uses a learned model 101 at a high speed. The learned model 101 is generated in advance by a computer dedicated to supervised machine learning, is built in the time lapse unit 50, and can be used via the DSP 56. The DSP 56 employs the configuration that can process the weighted operation processing suitable to machine learning over the multiple layers because there is a case where the DSP 56 relearns the learned model 101 as described later.

A memory card 59 is attachable to the memory interface 58. This memory card 59 records images captured by the camera module 51 as described later. The display interface 82 is connected with the display module 52, outputs an image signal to be displayed on the display module 52, and receives an input of a signal from a touch panel provided to the display module 52. The camera interface 81 is connected with the camera module 51, performs processing of exchanging signals for controlling the focus positions of the respective camera units CA1 to CA4 of the camera module 51 and receiving signals of images captured by the respective camera units CA1 to CA4, and further outputs signals to the LEDs turned on at a time of imaging. Note that the "interface" such as the memory interface 58 is abbreviated as the "I/F" in the drawings.

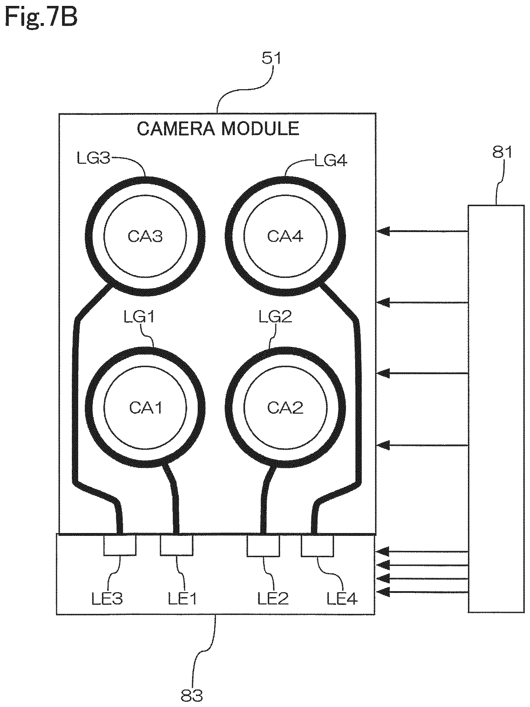

A light of the camera module 51 will be described with reference to FIG. 7B. As illustrated in FIG. 7B, an LED module 83 is attached to the camera module 51. The LED module 83 is provided with light emitting elements LE1 to LE4 that use illumination LEDs in association with the respective camera units CA1 to CA4. The light emitting elements LE1 to LE4 are each turned on and turned off according to a signal from the camera interface 81. The light emitting elements LE1 to LE4 are turned off by default. Note that the light emitting elements LE1 to LE4 may be light emitting elements such as organic ELs other than Light Emitting Diodes (LEDs). Furthermore, xenon lamps that are turned on at all times may be provided outside the time lapse unit 50, light of the xenon lamps may be brought into the time lapse unit 50 through optical fibers or the like, and guiding of the light to the camera module 51 may be turned on and off by a light switch.

Each of the light emitting elements LE1 to LE4 includes a light emission surface that faces toward the camera module 51, and this light emission surface is in contact with an end surface of each of light guide paths LG1 to LG4 formed in the camera module 51. The light guide paths LG1 to LG4 are so-called light guides, each include a core and a clad similar to the optical fiber, and guides light incident from the end surface to an annular terminal without leaking the light outside. The light of each of the light emitting elements LE1 to LE4 guided through each of the light guide paths LG1 to LG4 is emitted from the annular terminal to an imaging direction of each of the camera units CA1 to CA4, that is, a direction of the wells 95 in FIG. 6. A wavelength that does not influence the treatment egg 25 is selected for light emission from each of the light emitting elements LE1 to LE4. Note that the frequency of the light to be radiated on the well 95 is suitably a frequency at which a pronucleus that appears when the treatment egg 25 is fertilized is easy to see, that is, the frequency whose degree of absorption and reflection of light is significantly different at the pronucleus or a site other than the pronucleus.

### A2. Outline of Processing of Embryo Culture Device

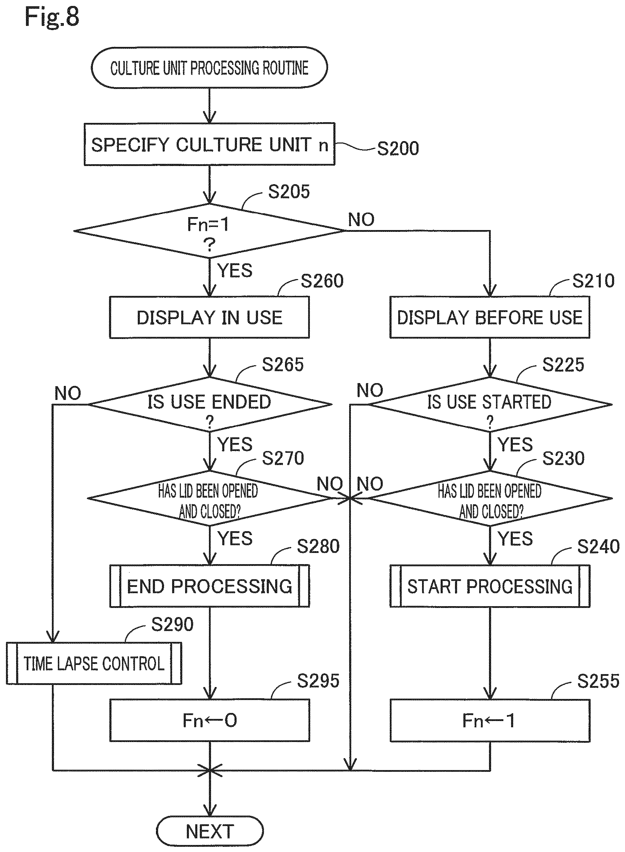

The above-described hardware configuration according to the first embodiment is the antecedent for the embryo culture device 10 to perform the following processing. According to the present embodiment, the control unit 60 of the embryo culture device 10 manages entire control, and the CPU 53 of the time lapse unit 50 manages imaging and display of the treatment egg 25. First, entire processing will be described with reference to FIG. 8. FIG. 8 is a flowchart illustrating a culture unit control routine. This processing is executed by the control unit 60.

The culture unit control routine illustrated in FIG. 8 is repeatedly executed at a predetermined interval when the embryo culture device 10 is powered on. When this processing routine is started, the culture unit for which processing is performed is determined first (step S200). In practice, a variable n for specifying the processing target culture units 11 to 22 is sequentially incremented between numerical values of 11 and 22 to specify the processing target culture unit. Note that the variable n is sequentially incremented, and, when the variable n reaches the value of 22, the variable n is set to the value of 11 at a time of next increment. That is, each of the culture units 11 to 22 becomes a processing target at a rate of one time in 12 times. When a number n of the processing target culture unit is determined in step S200, whether or not a flag Fn related to this culture unit n is a value of 1 is determined next (step S205). The flag Fn is reset to the value of 0 when the embryo culture device 10 is powered on.

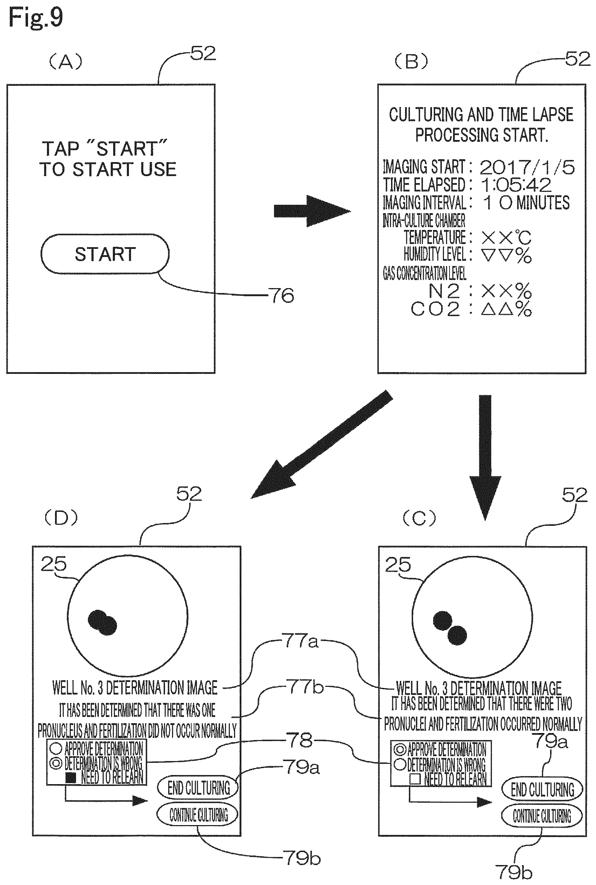

Hence, before each of the culture units 11 to 22 starts to be used, it is determined that the value of the flag Fn is not the value of 1, and pre-use display is performed next (step S210). The pre-use display is a processing of transmitting a signal to the time lapse unit 50 of the processing target culture unit n via the SIO 68, and displaying that this culture unit n is before use. Field (A) in FIG. 9 illustrates an example of display. Hence, all of the display modules 52 of the time lapse units 50 of the pre-use culture units display explanation "tap 'start' to start use", and touch screens display "start" buttons 76.

Next, whether or not the "start" button 76 of the screen has been tapped is determined (step S225), and, in a case where the "start" button 76 has been tapped, whether or not the lid has been opened or closed is determined next (step S230). The lid, that is, the time lapse unit 50 is opened and closed when a switch SWn associated with each culture unit n to be used is operated. The switch SWn is operated to open the time lapse unit 50 of the culture unit n to be used, and dispose in a culture chamber Rn the tray 91 accommodating the treatment eggs 25 in the wells 95, and the switch SWn is operated again to close the time lapse unit 50. Thus, it is determined that the lid has been opened and closed.

In the case where the lid has been opened and closed, it is determined that the tray 91 has been disposed in the culture chamber Rn, start processing (step S240) is executed, then the flag Fn is set to the value of 1 (step S255), and this processing routine moves to "NEXT", and is temporarily ended. In this regard, the start processing (step S240) means processing of placing the temperature, the gas concentration, and the like in the culture chamber Rn in a desired state, and then instructing the time lapse unit 50 to start time lapse processing. Furthermore, that the flag Fn is the value of 1 indicates that the culture chamber Rn is in use. When the start processing is executed, the display module 52 is caused to display information on an interior of the culture chamber. Field (B) in FIG. 9 illustrates an example of this display. In this example, "culturing and time lapse processing start" is displayed, and information of the current culture chamber Rn is displayed below this message. More specifically, a start time and a time elapsed for imaging, information related to imaging such as an imaging interval, environment in the culture chamber, i.e., information of the temperature and the humidity, and, moreover, information of the gas concentration and the like are displayed. The temperature and the gas concentration are detected by the unillustrated sensor. In addition to these pieces of information, information for specifying a patient of a treatment egg such as a chart number or the like may be displayed.

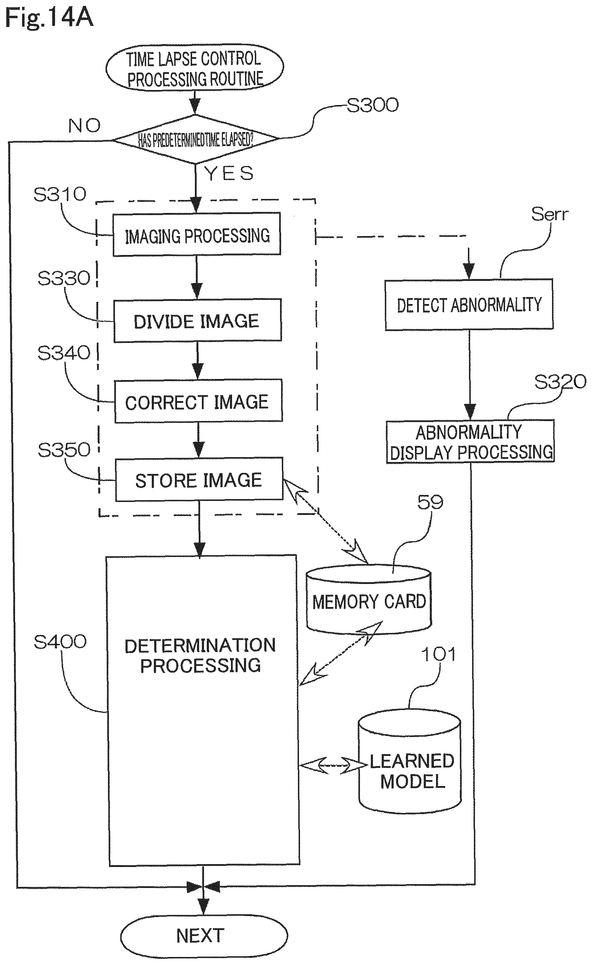

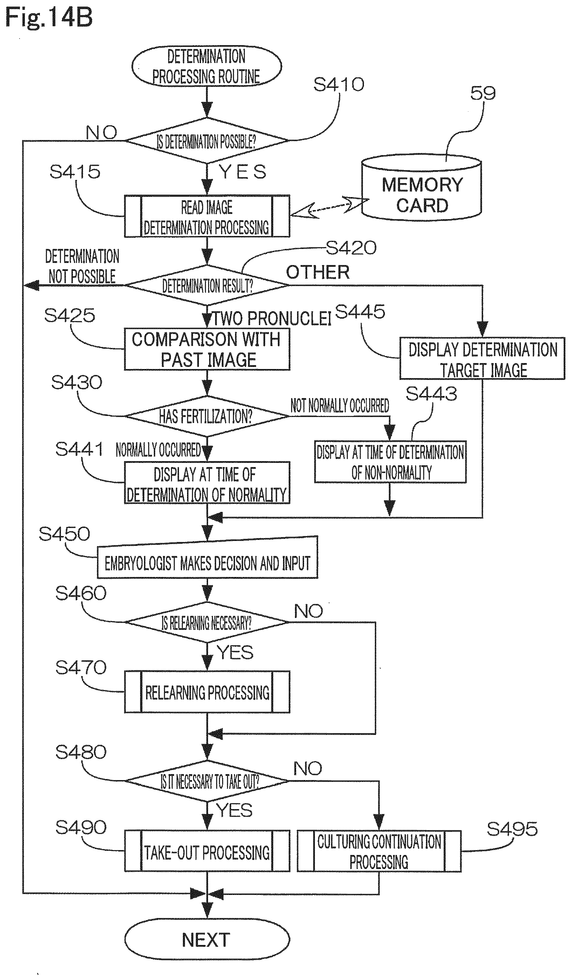

Culturing is started in this way, this processing routine is next activated when the flag Fn is set to the value of 1, the determination in step S205 is "YES" at the timing when the culture chamber Rn is specified in step S200, and processing moves to step S260. In step S260, the time lapse unit 50 is instructed to continue the time lapse processing. The time lapse processing executed by the time lapse unit 50 will be described later with reference to FIG. 14, yet, to put it simply, is processing of collectively imaging the plurality of respective wells 95 of the tray 91 in the culture chamber, dividing and storing images per well 95, individually determining a probability of fertilization of each treatment egg 25 in the well 95, and displaying a necessary image on the display module 52.

After the time lapse unit 50 is instructed to continue this time lapse processing, whether or not an instruction to end use has been accepted is determined (step S265). The instruction to end use can be obtained by an embryologist by operating the touch panel of the display module 52. When there is no instruction to end use, this processing routine moves to "NEXT" as is, and is temporarily ended. When there is the instruction to end use, whether or not the lid has been opened and closed is determined (step S270), and, after it is confirmed that the lid has been opened and closed, end processing (step S280) is executed. The end processing is processing of ending, for example, temperature control in the culture chamber Rn. Then, the flag Fn is set to the value of 0 (step S295), and this processing routine is temporarily ended.

### A3. Learned Model Generation Processing:

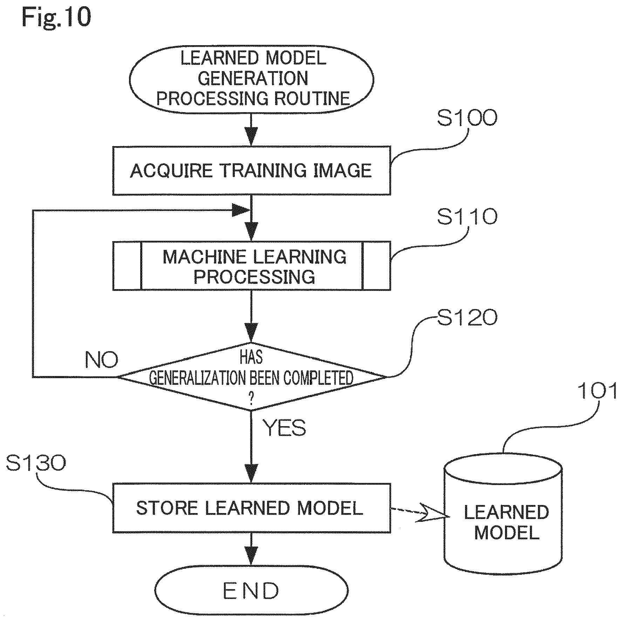

As described above, the time lapse unit 50 according to the present embodiment includes the learned model 101, and performs image recognition via the DSP 56. This learned model 101 is generated in advance by another computer. This generation processing will be briefly described. FIG. 10 is a flowchart illustrating one example of processing of generating the learned model 101. This processing is performed by another unillustrated computer for learning. This computer for learning can use a multilayer neural network that performs supervised learning.

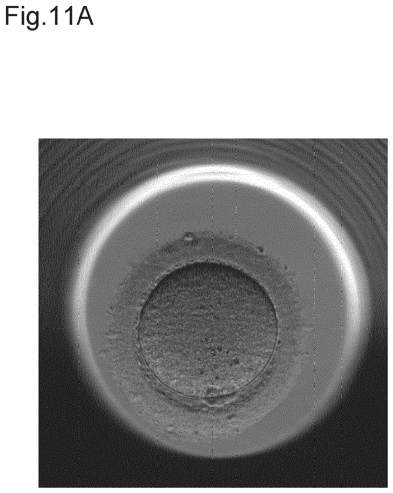

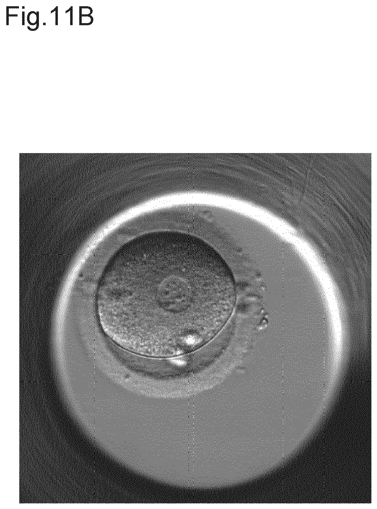

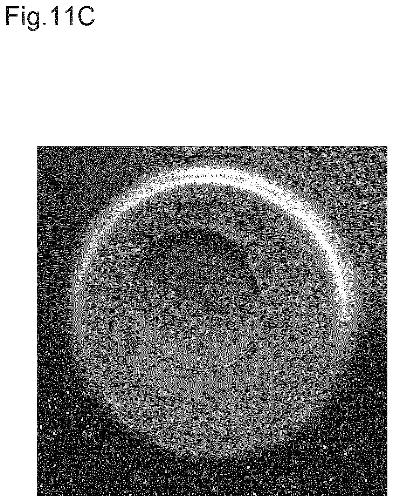

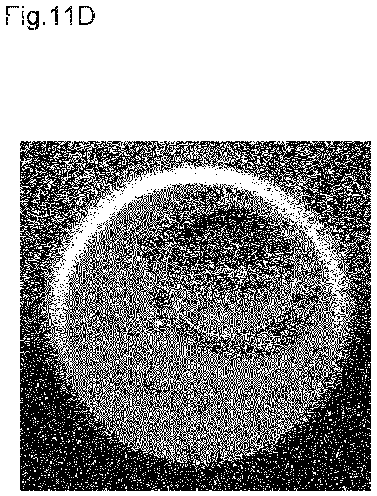

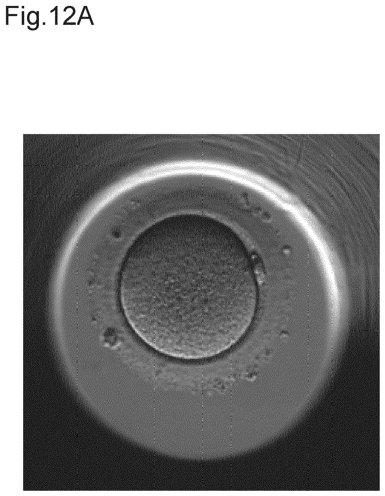

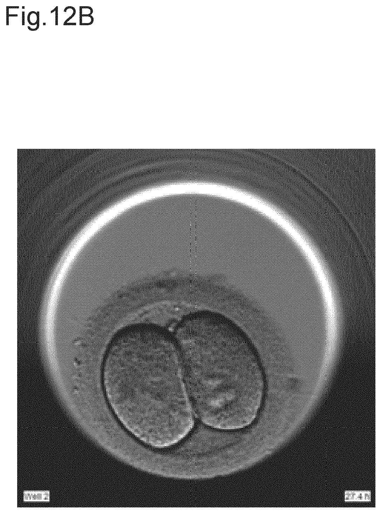

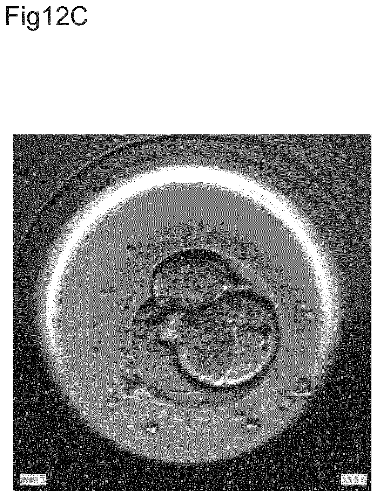

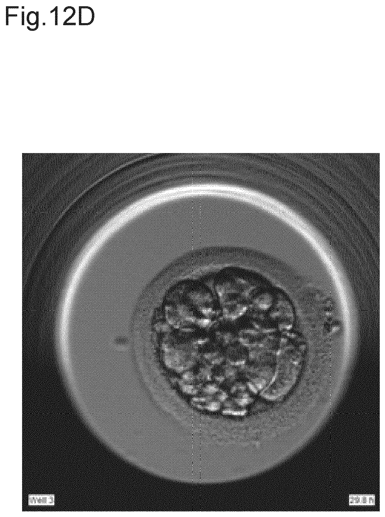

When this processing is started, the computer for learning acquires a supervised image first (step S100). The supervised image is an image that is attached a label indicating an image and what contents the image has. FIGS. 11A to 11D and FIGS. 12A to 12D illustrate examples of such an image. FIG. 11A illustrates an image attached a label A indicating an egg from which the pronucleus has not been confirmed. FIG. 11B illustrates an image attached a label B indicating an egg from which one pronucleus has been confirmed. FIG. 11C illustrates an image attached a label C indicating an egg from which two pronuclei have been confirmed. FIG. 11D illustrates an image attached a label D indicating an egg from three pronuclei have been confirmed. Furthermore, FIG. 12A illustrates an image attached a label E indicating an embryo in a state where cleavage after insemination processing is not started. FIG. 12B illustrates an image attached a label F indicating an embryo that has directly transitioned to a state where the number of blastomeres is two during cleavage after insemination processing. FIG. 12C illustrates an image attached a label G indicating an embryo that has transitioned to a state where the number of blastomeres is three during cleavage after insemination processing. FIG. 12D illustrates an image attached a label H indicating an embryo that has transitioned to a chaotic state during cleavage after insemination processing. Although not illustrated, an image attached a label indicating that the number of times of cleavage is four to eight, or an image attached a label indicating an embryo in which a blastocyst has been formed may also be included. In step S100, several hundreds to several thousands of multiple images attached these labels are acquired.

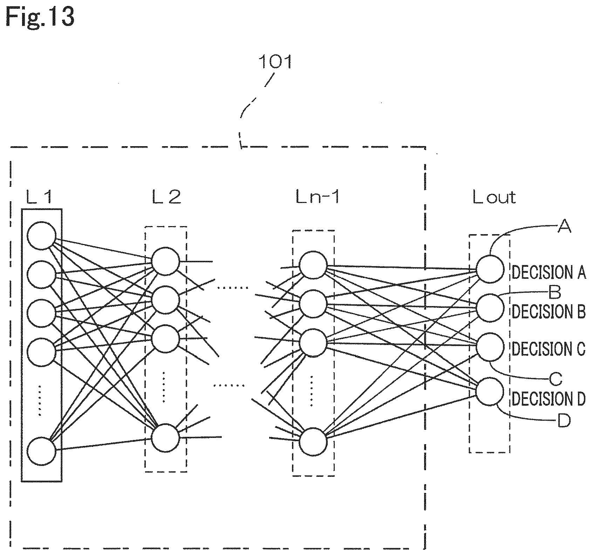

Next, machine learning is performed using this image (step S110). Machine learning is performed using a multilayer neural network as illustrated in FIG. 13. In FIG. 13, cuboids L1 to Ln-1 indicate respective layers of n-1 layers of a CNN. White circles indicate nodes of the neural network. The Convolutional Neural Network (CNN) is used in the present embodiment. However, other types of neural networks such as a capsule network type, a vector neural network type, and the like may be used.

The first layer L1 is provided with a plurality of nodes to which image data of the supervised image acquired in step S100 is input. In the present embodiment, a gradation value of each pixel of an image of a predetermined resolution is input data to each node of the first layer L1, and a label attached to this image is output as final output data from the final output layer Ln-1. Although the image to be input may be one image captured at a point of time when a predetermined time passes after one treatment egg is treated, two images of the same treatment egg captured with a random time interval apart from each other may be an image to be input as a set and learned. Furthermore, three or more images may be used. In the present embodiment, each image is individually learned.

The gradation value of each pixel of the image to be input is normalized to a value equal to or more than 0 and equal to or less than 1. A gradation value of a monochrome image is an input value in the present embodiment. However, each color gradation value of a color image of the RGB format can be also used. In a case where a color image is used, a neural network for training is provided for each of RGB, intermediate output data is output for each of RGB from each final layer of each neural network, and these three items of data may be collectively the entire final output layer Ln-1.

An output of each node of the first layer L1 is applied a weight, and is connected to a node of a next stage, that is, the second layer. The same applies to the second layer L2 and subsequent layers up to an n-2th layer. Due to the weight between the respective nodes between the respective layers, a result of the final output layer Ln-1 differs. By repeating work of correcting a weight between the respective nodes such that this result approximates training data (label) prepared in advance, learning is completed in the end, and the learned model 101 is generated. The weight is easily corrected by, for example, obtaining a loss function. More specifically, output values of the respective nodes (here, A to H in accordance with the types of the labels) of the final output layer Ln-1 are evaluated by the loss function. In a case where, for example, training data is for the image in FIG. 11A, a difference (learning error) from an actual output is specified by the loss function assuming a state where the node A takes a value of 1 and the other nodes B to H take values of 0. Then, the weight between the respective nodes is corrected. Work of obtaining a learning error again by the loss function after correction, finding a difference (differential value) from a previously obtained learning error, determining whether or not the learning error has been reduced as a result of weighting, and further correcting the weight is repeated for all acquired images. Note that, as the number of types of labels increases, the number of nodes in the final output layer may also be increased.

The following several functions are known as the loss function for obtaining a learning error. Some loss functions can be also adopted. One example is a cross entropy error. The loss function is calculated for all or part of data included in training data. The loss function of one learning is expressed by an average or a sum of differences from the training data. When the learning error of one learning is obtained, the weight between the respective layers is corrected by a known optimization algorithm. The optimization algorithm is, for example, stochastic gradient descent.

When the weight is corrected a predetermined number of times for all items of training data, whether or not generalization of a training model has been completed is determined (step S120). As for generalization, the degree of a difference between an input and an output of the training data to and from the training model is acquired, and, when the degree of the difference is less than a predetermined threshold, it is determined that generalization has been completed. Until it is determined that generalization is completed, machine learning processing (step S110) is repeated. Hence, until generalization is completed, processing of correcting the weight is repeated, and an operation of a learning error that uses an error function, and correction of the weight are repeated.

Each node of the final output layer Ln-1 is associated with a label of an identification target image. An output value of each node of the final output layer Ln-1 in a case where an image of training data is given is normalized such that a sum of the output values is the value of 1. That is, although, when a certain image is input, each node of the final output layer Ln-1 outputs a value corresponding to the property of the image, if learning is appropriately performed, the output value of the node associated with the label of this image takes the largest value. Consequently, in a case where learning is performed at an ideal level, when each training data is given, an output of one node of the final output layer Ln-1 takes the value of 1, and other outputs take the values of 0, so that it is possible to identify the label of the image. Nonetheless, as people's determination may make decision mistakes, each node of the multilayer neural network takes a value within a range large than the value of 0 and less than the value of 1 no matter how many times learning is repeated. Hence, the node whose output value is a maximum value among the respective nodes of the final output layer Ln-1 is assumed that a determination result, that is, the image indicates that this node is associated with the label. Whether or not learning is appropriately performed depends on a neural network structure and how high the quality of prepared supervised data is. Learning is performed using several hundreds of images in the present embodiment. However, it is preferable to use much more images.

When it is determined that learning has been completed in this way and generalization has been completed, processing of storing the learned model 101 is performed next (step S130). The learned model 101 is generated by a dedicated computer, is temporarily stored in this computer for learning processing, yet is subsequently transferred to the time lapse unit 50 in the embryo culture device 10. The time lapse unit 50 stores the learned model 101 such that the learned model 101 can be referred to via the DSP 56 as illustrated in FIG. 7A. Note that the learned model 101 is built in the time lapse unit 50 in the present embodiment, yet can be also provided on a cloud such that the learned model 101 can be referred to via a communication line. Alternatively, the learned model 101 may be stored in the control unit 60 illustrated in FIG. 4. After the learned model 101 is stored, the learned model generation processing routine moves to "END", and is ended. When an image of a treatment egg is input to the generated learned model 101, the multilayer neural network that has learned the weight of each layer can output which label the image is associated with, that is, whether or not the treatment egg captured in the image has a pronucleus, what the number of pronuclei is, how many eggs the treatment egg has been cleaved into, whether or not a blastocyst is formed, and the like.

When determination is made on a single image, which stage growth of a treatment egg is in is determined, and a state where a value of a node associated with a growth stage of the treatment egg is the largest among the respective nodes of a prepared output layer Lout is output. Consequently, by setting an output of a node whose value is a predetermined threshold or more to the value of 1 and an output of a node whose value is less than the threshold to the value of 0 in the final output layer Lout illustrated in FIG. 13, it is possible to output a result obtained by determining which stage the treatment egg is in. The value of each node may be output as is. When learning is performed by using a plurality of images captured at different timings as a set, and making a determination on the set instead of making a determination on images one by one, learning may be performed by preparing multilayer neural networks the number of which corresponds to the number of images included in the set and that are illustrated in FIG. 13, and further combining outputs of the plurality of neural networks. In this case, labels may be also created by taking into account a change over time such as whether or not a fertilized egg is a normally fertilized egg whose number of pronuclei has changed from 0 → 2 → 0 or whether or not a fertilized egg is an abnormally fertilized egg whose number of pronuclei has changed from 0 → 2 → 3.

Next, the time lapse processing will be described with reference to FIGS. 14A and 14B. FIG. 14A is a flowchart illustrating the time lapse control processing routine. This processing is processing that, when processing start of the time lapse processing is instructed in step S240 in FIG. 8, is repeatedly executed at the predetermined interval by the time lapse unit 50 until the end processing is performed in step S280. Furthermore, FIG. 14B is a flowchart illustrating details of determination processing (step S400) of the time lapse control processing. Details of the determination processing will be described later.

When the time lapse control processing illustrated in FIG. 14A is started, whether or not a predetermined time has passed since an image of a treatment egg is previously captured is determined first (step S300). The predetermined time described herein corresponds to the imaging interval illustrated in field (B) in FIG. 9. The predetermined time may be a much shorter time or longer time instead of 10 minutes illustrated in field (B) in FIG. 9. In a case where the predetermined time has passed since previous imaging (step S300: "YES"), imaging processing is performed (step S310). The plurality of wells 95 are collectively imaged, that is, the respective camera units CA1 to CA4 collectively image the four wells 95 present in the areas U1 to U4. The captured images are stored together with data of an imaging time in the memory card 59.

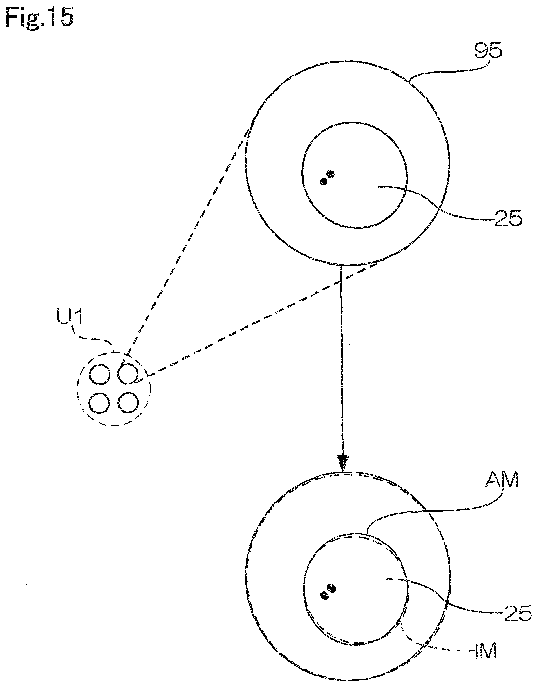

The imaging processing (step S310) is performed by simultaneously or sequentially driving the four camera units CA1 to CA4 provided in the camera module 51 of the one time lapse unit 50. As illustrated in FIG. 5, by causing the respective camera units CA1 to CA4 to operate, states of the treatment eggs 25 accommodated in the 16 wells 95 of the tray 91 are imaged. FIG. 15 illustrates how the states of the treatment eggs 25 are imaged. The camera units CA1 to CA4 image the areas U1 to U4, respectively, and the areas U1 to U4 include the four wells 95 in the present embodiment.

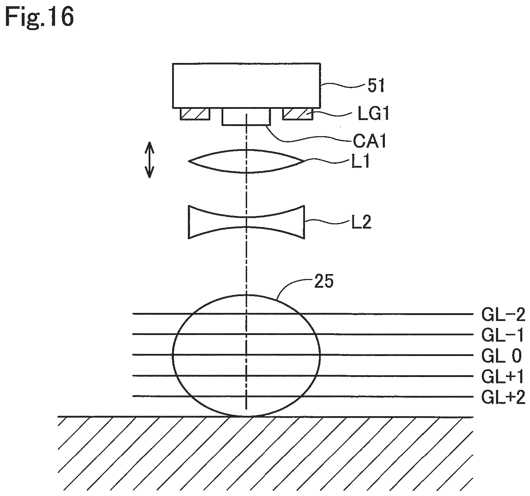

Each of the camera units CA1 to CA4 images the tray 91 from above. In this case, the camera units CA1 to CA4 image five images around the one well 95 while moving the focus positions. FIG. 16 illustrates how imaging is performed. Although FIG. 16 schematically illustrates lenses L1 and L2 outside the camera unit CA1, the lens L1 and L2 are actually built in the camera unit CA1. By moving at least one of the lenses L1 and L2 forward and backward along the optical axis, the focus position of the camera unit CA1 may change to a position GL+1 that is a on side distant from the camera unit CA1, a more distant position GL+2, a position GL-1 that is on a side closer to the camera unit CA1, and a closer position GL-2 with respect to a center position GL0. The size of the treatment egg 25 is approximately 100 to 150 µm in a case of an egg of a person, and therefore a difference between the respective positions is set to approximately 20 to 25 µm. In this case, a lens configuration of the camera units CA1 to CA4 has a shallow focal depth, and an imaging range at each focus position is adjusted to 25 to 30 µm. Each one of these camera units CA1 to CA4 captures five images. A plurality of images of different focus positions are captured because at which position of the treatment egg 25 that is a substantially spherical body a location of a pronucleus or the like in the treatment egg 25 that serves as a clue for determining fertilization is not known. Note that a lens configuration having a deep focal depth may capture a single image without changing the focus position in this way.

After the imaging processing, processing of dividing the captured image per well 95 is performed (step S330). Here, there are the respective four wells 95 in the areas U1 to U4 imaged by the respective camera units CA1 to CA4, and therefore the captured image is divided into four. Next, processing of correcting the divided images is performed (step S340). Correction is performed to remove distortion and dirt or debris from the images. The plurality of wells 95 are imaged at once, and therefore all of the wells 95 cannot be located right below the optical axes of the camera units CA1 to CA4. Hence, the image obtained by imaging each well 95 is distorted. In a case of a color image, there is also a case where aberration causes blur or color-tint.

Then, image correction (step S340) corrects such distortion, dirt or debris, and the like. FIG. 15 is an explanatory view illustrating an example of correction. When each one of the wells 95 in the area U1 is divided and taken out, the well 95 is not right below the optical axis, and therefore the outline of each well 95 formed in a true circle is distorted and does not become the true circle. Naturally, similar distortion occurs in the captured images of the treatment eggs 25, too. When the distorted image is corrected as indicated by broken lines in FIG. 15, the outline of each well 95 becomes the substantially true circle. The shape of the treatment egg 25 is also corrected likewise to the original shape. Such correction is performed not only to correct distortion of the shape, but also to remove color-tint due to chromatic aberration, a ghost caused by dirt or debris on a lens, and the like.

After image correction, if correction can be performed, processing of storing the image in the memory card 59 is performed (step S350). In this case, the image is stored per divided image. A tag for determining which one of the camera units CA1 to CA4 has captured each divided image may be attached as a file name or the like to each divided image to link. Furthermore, when each image is stored, only a corrected image may be stored, or the corrected image may be stored together with a pre-correction image or separately from a pre-correction image. Storage is not limited to the memory card 59, and each image may be stored on, for example, a server on a cloud. A storage destination is not limited to a memory card, and may be other storage media such as hard disks and SSDs.

During the above-described series of processing, that is, processing from imaging of the areas U1 to U4 by the camera units CA1 to CA4 to dividing, correcting, and then storing of each image, the CPU 53 monitors whether or not some abnormality has occurred (step Serr). Whether or not the abnormality has occurred is occasionally determined by interruption processing.

When it is detected that some abnormality has occurred from imaging to storage (steps S310 to S350) (step Serr), abnormality display processing (step S320) of displaying contents of an abnormality on the display module 52 is performed. In this case, the display module 52 functions as a presentation unit that presents an abnormality. The contents of abnormality are exemplified as, for example,
Light turn-on abnormality
Camera operation failure
Correction processing abnormality, and
Image division or storage abnormality
When these abnormalities occur, corresponding portions are marked and displayed. When overlapping abnormalities occur, a plurality of items are marked.

"Light turn-on abnormality" among the abnormalities refers to a case where each of the light emitting elements LE1 to LE4 of the above-described LED module 83 is not turned on, a case where light does not arrive due to an abnormality of the light guide paths LG1 to LG4, and a case where the wells 95 are not normally illuminated. In this case, the captured image becomes dark, and average brightness of an image does not fall within a predetermined range, so that it is possible to determine the abnormality. The camera operation failure refers to, for example, a case where the camera units CA1 to CA4 do not operate, and a case where light reception modules of a CCD and a CMOS for imaging do not operate. When an optical focus adjustment device and a shutter are provided, operation failures of these devices are also included. As for the light reception modules, the camera operation failure can be determined when, for example, a readout clock is not inputting normally. Note that, in the present embodiment, the camera unit CA1 is provided with a diffusion plate that uniformizes illumination light that is emitted from the light emitting elements LE1 to LE4, passes through the light guide paths LG1 to LG4, and illuminates the wells 95, and for example, a case where illumination light is not uniform due to an attachment direction of this diffusion plate or an attachment mistake of top and back sides of this diffusion plate is included in the light turn-on abnormality.

The correction processing abnormality refers to a case where correction for removing distortion and dirt or debris from a captured image is not normally performed. For example, the distortion is removed by inputting a gap from the optical axis or the like as a parameter in advance, and correcting a shape using this parameter, and, in a case where the shape of the well 95 is significantly different from the true circle as a result of correction, it is determined that correction processing has not been normally performed. Furthermore, although the processing of removing dirt or debris is normally performed by removing approximately one or two isolated dots on a screen, in a case where the number of isolated dots is a predetermined number or more, it is determined that the correction processing has not been normally performed.

The division abnormality of the image division or storage abnormality refers to, for example, a case where, when an image obtained by imaging the plurality of wells 95 is divided, and division processing of the image is normally ended, or an imaging center of the camera unit CA1 or the like is deviated and each well 95 does not fit in each divided image as a result of division. The divided image storage abnormality refers to, for example, a case where, when a file of a divided image is stored in the memory card 59 or the like, the file cannot be normally stored due to an insufficient capacity, a failure, or the like of the memory card 59.

When there is no abnormality in particular after the above processing (steps S330 to S350) is ended, determination processing (step S400) of determining a state of the treatment egg is executed. According to the determination processing (step S400), an image stored in the memory card 59 is read, and the state of the treatment egg is determined from the read image using the learned model 101. FIG. 14B illustrates details of this determination processing.

When the determination processing starts, whether or not a timing at which determination of fertilization or cleavage can be made has come is determined (step S410). Determination of fertilization means determining whether or not the treatment egg 25 subjected to fertilization treatment has been fertilized. Fertilization generally succeeds until approximately eight hours pass at the earliest, approximately 22 hours pass on average, or 36 hours pass at the latest after the fertilization treatment. The treatment egg 25 subjected to the fertilization treatment is quickly accommodated in the well 95 of the tray 91 after the treatment, and is placed in one of the culture units 11 to 22. Therefore, when approximately six hours pass after the time lapse unit 50 starts imaging, determination in step S410 is "YES", that is, determination of fertilization is possible. This time may be made much shorter, and determination of fertilization may be possible immediately after the trays 91 are accommodated in the culture chambers R11 to R22. In this case, the determination in step S410 is unnecessary.

When it is determined that determination is possible, determination processing is performed next (step S415). This processing is processing of reading the image of the treatment egg 25 divided per well 95 and stored in the memory card 59, and determining in what state the treatment egg captured in the image is. As illustrated in FIGS. 11A to 11D and FIGS. 12A to 12D, the treatment egg may undergo some states until fertilization and some states until formation of a blastocyst. As already described above, these states also include a state where fertilization does not necessarily normally occur, and a state where cleavage does not normally proceed. The learned model 101 has learned both of the image showing the state of progress of normal fertilization or cleavage and an image showing a state that is not normal, so that the DSP 56 can perform determination processing (step S415) of determining in what state the treatment egg captured in the acquired image is using the learned model 101. At this time, in the present embodiment, the acquired image is a latest captured image. This is because supervised machine learning is performed using a single image in the present embodiment. Hence, when determination is made using a new image, determination is made using one image likewise.

By contrast with this, determination may be made on the same treatment egg using a plurality of images captured immediately after treatment, that is, using time lapse images. Alternatively, determination may be made using a combination of two images captured with a random time interval apart from each other. Note that the random time interval of learning and the random time interval of determination may be the same or may be different. For example, images at a time of learning are images captured every five minutes, and images at a time of determination may be images captured every 10 minutes. A phenomenon such as fertilization and cleavage that proceeds inside a treatment egg chronologically varies, and therefore adjusted imaging time intervals do not have a special meaning.

Processing of determination based on such an image can be achieved in real time by using a high speed processing function of the DSP 56 illustrated in FIG. 7A. After the determination processing (step S415) based on the image is executed, processing of deciding subsequent processing based on the determination result is performed (step S420). The determination result is roughly classified into
·That determination is impossible,
·Determination that two pronuclei have been confirmed, and
·Determination that the treatment egg is in other states.
That determination is impossible means a case where any output of the final output layer Lout illustrated in FIG. 13 does not exceed a threshold (e.g., a value of 0.5), and in which stage the treatment egg is cannot be determined. Such a result may also occur in a case where some abnormality that is not learned occurs in the treatment egg, and, in addition, in a case of an image of a treatment egg before the pronucleus appears or an image of an embryo that has not reached a fourth cell stage. In this case, any processing is not performed in particular, and this determination processing routine moves to "NEXT", and is temporarily ended. Note that, at this stage, an image captured once may be displayed on the display module 52 to ask for an embryologist to make a determination. Furthermore, an option, that is, whether or not a treatment egg corresponds to a poor embryo may be provided to the determination result in step S420, and, display that a determination processing target image has been determined to belong to a poor embryo learned in advance when this determination is made, and accept an instruction to, for example, stop culturing.

On the other hand, when the determination result obtained by using the learned model 101 indicates that the two pronuclei have been confirmed in a treatment egg, processing of reading a determination result of a past image of this treatment egg is subsequently performed (step S425). The image of each treatment egg is stored together with the determination result of each treatment egg, and therefore it is easy to read a past image and the determination result of the past image. After the past image and the determination result of the past image are read, whether or not fertilization has normally occurred is determined. In a case where it is determined that fertilization has normally occurred, the display module 52 shows display at a time of determination of normality (step S441). On the other hand, in a case where it cannot be determined that fertilization has normally occurred, the display module 52 shows display at a time of determination of non-normality (step S443).

Whether or not fertilization normally occurs can be determined by checking a change in the number of pronuclei in the image. In a normally fertilized egg, two pronuclei generally appear within 22 hours after fertilization. In the present embodiment, according to the determination processing, whether or not a treatment egg is fertilized by analyzing two images acquired at different times. That is, when an image in which the number of pronuclei is two is detected, a past image is read and matched with the image to determine that fertilization normally occurs when the number of pronuclei directly changes from zero to two. "Directly" means that a pronucleus does not undergo a state where the number of pronuclei is one. On the other hand, even when an image in which the number of pronuclei is two is detected, in a case where a past image is read and matched with the image, and the number of pronuclei does not directly change from zero to two such as a case where it is determined from a first image that the number of pronuclei is two, or a case where the number of pronuclei changes from 0 → 1 → 2, it is determined that fertilization does not normally occur. This determination may be made by other methods. In a case where, for example, the learned model 101 has learned to handle a plurality of chronologically stored images, the plurality of images may be given to directly determine using the learned model 101 whether or not fertilization normally occurs.

Field (C) and field (D) in FIG. 9 illustrate examples of display according to determination in step S430. In this example, the latest image of the treatment egg 25 for which determination of fertilization has been made, number display 77a indicating what number well 95 this treatment egg is placed, a message 77b indicating a determination result, and various display 78 for instructing subsequent treatment are displayed on the display module 52. Field (C) in FIG. 9 illustrates a case where determination of "normal fertilization" is made using the learned model 101. In this case, a message "it has been determined that fertilization normally occurred" is displayed as the message 77b. Furthermore, a radio button for alternatively instructing whether this determination is approved or wrong, and a check box for designating whether or not to relearn the learned model 101 when it is determined that this determination is wrong are displayed as the various display 78. Furthermore, a screen displays a "culturing end" button 79a that instructs ending culturing as subsequent treatment of this treatment egg, and a "culturing continue" button 79b that instructs continuing culturing.

On the other hand, display (step S443) in a case where determination of fertilization (step S430) is that fertilization has not normally occurred shows the message "it has been determined that "fertilization did not normally occur" is displayed as the message 77b as illustrated in field (D) in FIG. 9. Furthermore, the radio button for alternatively instructing whether this determination is approved or wrong, and the check box for designating whether or not to relearn the learned model 101 when it is determined that this determination is wrong are displayed as the various display 78. Furthermore, the screen displays the "culturing end" button 79a that instructs ending culturing as subsequent treatment of this treatment egg, and the "culturing continue" button 79b that instructs continuing culturing. The latest image on which determination has been made is displayed in these examples. However, when determination is made in comparison with a previous image, a plurality of determination target images may be displayed collectively. In this case, the images may be aligned and displayed on the display module 52, or may be switched and displayed one by one by, for example, clicking a mouse button. Furthermore, when there is a part recognized as particularly specific characteristics during determination, this part may be enlarged and displayed.

In a case where it is determined in step S420 that the image for which determination has been made using the learned model 101 is an image other than an image of pronuclei the number of which is other than two, determination target image display (step S445) is performed. This determination target image display is processing of displaying an image of a treatment egg, a well number of this image, and a button for accepting ending culturing or continuing culturing as an instruction of subsequent treatment similar to fields (C) and (D) in FIG. 9 when the image for which determination has been made is a specific image of pronuclei the number of which is other than two such as an image of three pronuclei, an image of a second cell stage after cleavage starts, an image of a fourth cell stage, or an image in which formation of a blastocyst has been confirmed, or when an output of one of nodes becomes a threshold or more according to the learned model 101.

When the determination processing target image is determined as an image of cleavage such as an image of a second cell stage, a fourth cell stage, an eighth cell stage, or the like, a previous image of this image may be read to determine whether or not cleavage normally occurs. In a case where, for example, progress of normal fertilization (embryo) → second cell stage → fourth cell stage occurs, it may be determined that the embryo is a good embryo, and, in a case where a third cell stage appears during this stage, it may be determined that the embryo is not a good embryo, and a result of this determination may be displayed. Note that relearning is not performed in cases other than fertilization determination in the present embodiment. However, a button for accepting an instruction for performing relearning may be displayed to perform relearning according to the instruction. Relearning will be described later.

After one of the above-described display processing (steps S441, S443, and S445) is performed, processing of accepting decision of the embryologist and an input based on this decision is performed (step S450). This processing is processing of causing the embryologist to make decision looking at display on the display module 52, and accepting the input based on this decision. When, for example, the device determines that there are two pronuclei and fertilization normally occurs, the embryologist accepts a result of this determination, and decides ending or continuing fertilization. In this case, the instruction of the embryologist's decision is accepted as the input by pushing the button 79a or 79b. Alternatively, the embryologist may decide that the image for which it has been determined that there have been two pronuclei and fertilization has normally occurred is not an image of the two pronuclei. In this case, the determination made by the device is denied, and the embryologist's instruction for performing relearning is accepted as an input.

As for cleavage, if in which cell stage embryo transfer is performed is determined in advance according to a patient's state, the embryologist decides to end culturing at a timing of this cell stage such as the second cell stage, the fourth cell stage, or a blastocyst formation period. In this case, the embryologist's instruction is accepted as an input. In a case where the timing of embryo transfer has not come, the instruction to continue culturing is accepted as an input.

After this embryologist's decision and the instruction based on this decision, whether or not "relearning" is necessary is determined next (step S460). In a case where the embryologist determines that determination made using the learned model 101 is wrong, and tick the check box of "relearning required" of the various display 78, relearning processing is performed (step S470). Relearning is processing of adding as a new image an image for which relearning is necessary even though the image had been learned once, and correcting or adding a weight between layers of the learned model 101. This processing may be achieved by performing again the learned model generation processing described with reference to FIG. 10, or may be achieved by a transfer learning method for performing relearning by adding a new layer to the already generated learned model 101. Furthermore, other relearning methods may be adopted. Note that relearning may be performed inside the time lapse unit 50 using the DSP 56, yet may be performed in a dedicated circuit of the embryo culture device 10 other than the time lapse unit 50. Alternatively, relearning may be performed using a learning dedicated computer on a network using a communication line, and a result of relearning may be accepted via the communication line.

After determination (step S460) on whether relearning is necessary or unnecessary and relearning (step S470) in a case where relearning is necessary are performed, whether or not it is necessary to take out the tray 91 is determined next (step S480). In a case where the input from the embryologist is "end culturing", it is determined to take out the tray (step S460: "YES"), and processing of taking out the tray 91 is performed (step S490). In a case where the input from the embryologist is "continue culturing", processing of continuing culturing as is is performed (step S495). The processing of continuing culturing continues processing of keeping environment in the culture chamber Rn in a state suitable for culturing. After these processing is ended, this processing routine moves to "NEXT", and is ended.

The embryo culture device according to the above-described first embodiment includes the time lapse unit 50 provided to each of the 12 culture chambers R11 to R22, images the treatment egg 25 accommodated in each of the plurality of wells 95 of the tray 91 at the predetermined interval, applies the learned model generated in advance by supervised machine learning using this image, makes a determination of growth of the treatment egg, that is, determination of whether or not fertilization has normally occurred in this case, and reads from the storage unit an egg image of the treatment egg having been determined to satisfy the condition set in advance according to a result of the determination. Consequently, it is possible to determine whether or not fertilization has normally occurred or in which cell stage an embryo is using images captured without relatively moving the treatment eggs 25 accommodated and cultured in the wells 95 with respect to the camera units provided to the display module 52, so that it is possible to reduce fluctuation of environment that is disadvantageous for the treatment eggs that are being cultured. Moreover, it is possible to accurately grasp situations of the treatment eggs that are being cultured, and take a necessary measure. In this regard, it is possible to accurately perform treatment such as fertilized egg cryopreservation or embryo transfer following an end of culturing. Alternatively, it is also possible to determine continuing culturing, and further grow treatment eggs.

In the present embodiment, each one of the camera units CA1 to CA4 is caused to image the plurality of wells, and this image is divided into an image of each well 95 and stored. Consequently, it is possible to reduce the number of times of imaging, and shorten an imaging interval. Furthermore, each divided image is corrected, so that it is possible to make a determination of fertilization or cleavage based on the corrected image, and more accurately make a determination of fertilization or cleavage. Furthermore, the image divided per well 95 is stored, so that it is possible to read only the image of the well 95 of interest. Consequently, it is possible to reduce a probability that the treatment egg 25 of the another well 95 is used as a fertilization determination target or the like by mistake.

According to the above embodiment, in a case where an abnormality occurs during one of imaging of the wells 95 by the camera units CA1 and CA2 (FIG. 14 and step S310), subsequent division of an image (step S330), correction of the image (step S340), and storage of the image (step S350), this abnormality is detected, and displayed on the display module 52. Consequently, it is possible to immediately learn of the abnormality when some abnormality occurs during acquisition of the image of the well 95. Hence, it is possible to avoid that it is not possible to obtain the image of the treatment egg 25 in the well 95 over a long period of time and make a determination of fertilization, or it is difficult to make a determination of fertilization. A user who has learned of the abnormality can take measures of, for example, repairing the embryo culture device 10, replacing the camera unit, exchanging the time lapse unit 50 itself, or moving the tray 91 to the other culture chambers R11 to R22, and continue imaging, dividing, correcting, or storing the treatment egg 25.

According to the above embodiment, it is not necessary to further move the tray 91 or the imaging device (such as the camera) to image the treatment egg 25. Consequently, the embryo culture device 10 not only can be made smaller, but also does not include a movable part, so that it is possible to enhance reliability of the embryo culture device 10. Furthermore, neither vibration nor noise occurs accompanying transportation of trays and cameras. Furthermore, it is possible to shorten the interval to image the treatment egg 25. In a case where the system that moves and images the tray 91 takes, for example, m minutes for moving and imaging the tray, 12×m minutes are required to move and image all of the 12 trays 91. That is, the interval cannot be made shorter than this time of 12×m minutes. By contrast with this, the embryo culture device 10 according to the present embodiment can shorten imaging intervals to imaging intervals of the camera units CA1 to CA4 as much as one likes. It is also possible to continuously perform imaging.

Furthermore, according to the present embodiment, the one time lapse unit 50 is prepared for the one tray 91 accommodated in one of the culture chambers R11 to R22, this time lapse unit 50 is provided with the display module 52, and the image of the treatment egg 25 in the tray 91 accommodated in one of the culture chambers R11 to R22 is displayed thereon. Hence, there is also an advantage that the image displayed on the display module 52, and the tray 91 in the state where the switch SWn is operated and the time lapse unit 50 is opened have a one-to-one relationship, and the relationship between the image and the treatment egg is very clear.

Furthermore, according to the present embodiment, the time lapse control processing is performed per time lapse unit 50, so that it is possible to individually set time lapse processing such as an interval for imaging, a format of an image to display, and the like per time lapse unit 50 of each of the culture units 11 to 22. In a case of, for example, a treatment egg whose development after insemination treatment is of interest in particular, it is also easy to take measures of shortening the imaging interval, or asking for the embryologist to make a determination when determination of a state of fertilization or cleavage is made.

Furthermore, the time lapse unit 50 according to the present embodiment each includes the memory card 59, and divides and records images captured at the predetermined interval per well 95. Consequently, an image used to make a determination on a treatment egg cultured in this culture chamber Rn is not taken as an image of another treatment egg by mistake. If necessary, captured images can be, for example, managed, stored, and discarded per memory card, and easily handled.

Although the imaging processing performed at the predetermined interval and the determination processing related to growth of treatment eggs in the processing illustrated in FIG. 10 have been described with reference to one flowchart for ease of description, both of the processing may be performed by separate processing routines in an asynchronous manner.

A display time in a case where imaging and determination are performed as a series of processing will be studied. The 2×2×4 (16 in total) wells 95 are provided in the tray 91 placed in the one culture chamber Rn, and the 16 treatment eggs can be accommodated at maximum. The one time lapse unit 50 can simultaneously image these 16 wells 95 using the four camera units CA1 to CA4. Here, a case will be studied where, while the treatment eggs accommodated in these 16 wells 95 are sequentially displayed on the display module 52, the above determination is made and a determination result is displayed. Assuming that the imaging interval is 10 minutes, each well 95 can be displayed for approximately 50 seconds. Consequently, the embryologist can learn the number of the well 95 accommodating the treatment egg that is the determination target. Furthermore, it is also possible to determine the end of culturing. Note that the imaging interval may be increased. When the number of wells to be imaged at once is small, the interval may be shortened. The number of wells to be imaged here may be the number of wells in which treatment eggs are actually disposed, or a maximum number of wells.

Performing insemination treatment simultaneously is rare. Therefore, even when there are the 12 culture units 11 to 22 in the embryo culture device 10 according to the present embodiment, the fertilized eggs are not necessarily displayed simultaneously in the 12 culture units in the entire embryo culture device 10. Consequently, in a case where only the treatment eggs for which determination of fertilization and cleavage has been made are displayed on the display module 52, even when there are the 12 culture units 11 to 22, the embryologist can confirm fertilization and cleavage looking at an image of this fertilized egg to be sequentially displayed, take out the tray 91, and perform subsequent treatment.

### B. Other Embodiment:

While the time control processing is executed by the time lapse unit 50 alone in the above-described embodiment, entire processing including the time lapse control processing, too, may be performed by the control unit 60. Furthermore, a device may be configured to entirely automate accommodation of the treatment eggs 25 in the wells 95 of the trays 91 to delivery of the fertilized eggs that have been fertilized to next treatment.

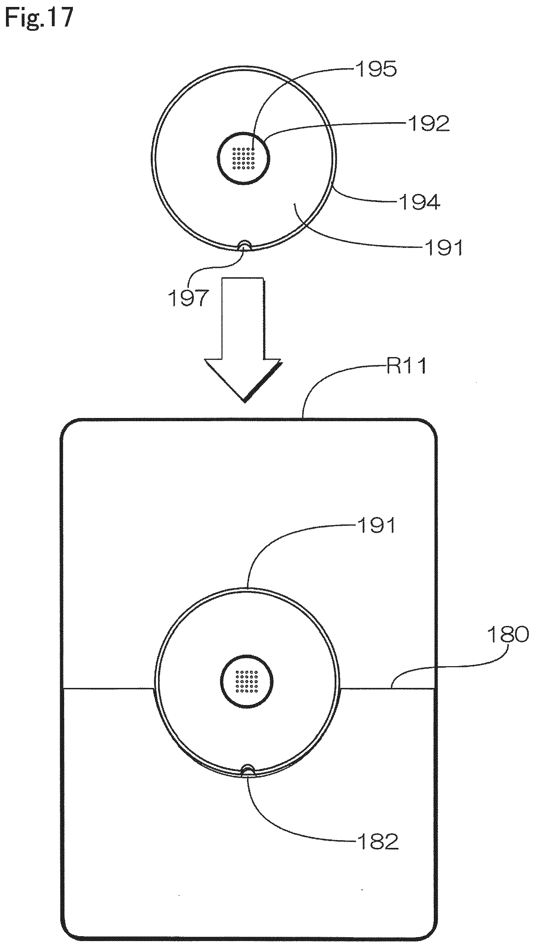

The four camera units CA1 to CA4 have been prepared per one time lapse unit 50 in the above embodiment. However, the number of camera units per time lapse unit 50 is random. The number of the wells 95 imaged by the camera units, that is, the number of treatment eggs to be imaged at once may be one, yet may be a random number equal to or more than two. Furthermore, an arrangement of the wells in the tray 91 is also random. For example, p×q wells may be aligned in an array pattern (one of p and q is a positive integer equal to or more than two). FIG. 17 is an explanatory view illustrating a dish 191 including 5×5, that is, 25 microwells 195 in total accommodating treatment eggs. While an upper part in FIG. 13 illustrates the dish 191, this dish 191 is accommodated at an accommodation portion of the culture chamber R11 in a state where the treatment eggs are accommodated in the microwells 195. The dish 191 has a cylindrical shape whose diameter is approximately 35 mm to make handling easy, and includes at the outer circumference an outer circumferential wall 194 whose height is approximately 10 mm. Furthermore, the dish 191 is provided with a circular partition wall 192 of approximately 2 mm to surround the microwell 195. An inside of this partition wall 192 is filled with a culture medium. The treatment egg is accommodated in the microwell 195 filled with the culture medium.

By so doing, at a time of handling of the dish 191, it is easy for the embryologist to carry the dish 191, and the culture medium only needs to fill small sections partitioned by the partition wall 192, so that the culture medium is not wastefully used. Furthermore, the 25 microwells 195 that accommodate the treatment eggs are accommodated in a several millimeter square, so that one camera unit can easily image the 25 microwells 195. When an imaging area is narrow, it is possible to increase resolution of an image per treatment egg, and enhance accuracy of determination of fertilization or the like.

The dish 191 illustrated in FIG. 17 includes a recess part 197 at one portion on an outer side of the outer circumferential wall 194. This recess part 197 is used to fit to a protrusion 182 provided to a holding frame 180 that holds the dish 191 and position the dish 191 at a predetermined position when the circular dish 191 is accommodated in the culture chamber R11. The holding frame 180 is formed in a shape that can accommodate an approximately half circumference of the dish 191, and the dish 191 is stably held at a predetermined position in the culture chamber R11. Note that the dish 191 is not limited to the circular shape, and may have other shapes such as a rectangular shape, an elliptical shape, and a trapezoidal shape. Furthermore, the partition wall 192 is not limited to the circular shape, and may have other shapes such as a square shape. Furthermore, the entire dish 191 may be filled with the culture medium without providing the partition wall 192.

In addition, the microwells 195 may be aligned in other alignment patterns such as an annular pattern. When necessary resolution can be obtained, any number of treatment eggs may be imaged at once. Contents and a method of correction and image division may be changed based on arrangement of the wells 95 and the microwells 195. Furthermore, a plurality of camera units the number of which is smaller than the number of trays may be provided, may move within the time lapse unit 50 and share imaging of the plurality of trays between the camera units.

The one tray 91 is disposed in the one culture chamber Rn in the above embodiment. However, two or more trays may be disposed in the one culture chamber Rn. In this case, the one time lapse unit 50 may be associated with the plurality of trays, and the one time lapse unit 50 may be associated with the one tray.

The time lapse unit 50 and the camera module 51 are also disposed as a lid at the upper part of the culture chamber Rn in the above embodiment. However, the lid of the culture chamber Rn may be provided separately from the camera module 51. Furthermore, the camera module 51 can be also disposed on a bottom surface or a side surface of the culture chamber Rn. In a case where the camera module 51 is disposed on the bottom surface, the bottom surface of the culture chamber Rn may be made of a material such as transparent glass, the camera unit CA1 or the like may be disposed below the bottom surface, and the treatment egg in the well 95 of the tray 91 may be imaged. Generally, the treatment egg sinks to the lowermost part in the well 95, and therefore it is easy to adjust the focus of the camera unit to the treatment egg by imaging the treatment egg from below the floor surface. In this case, only the display module 52 may be disposed as the lid at the upper part of the culture chamber Rn, or the display module 52 may be provided at another place.

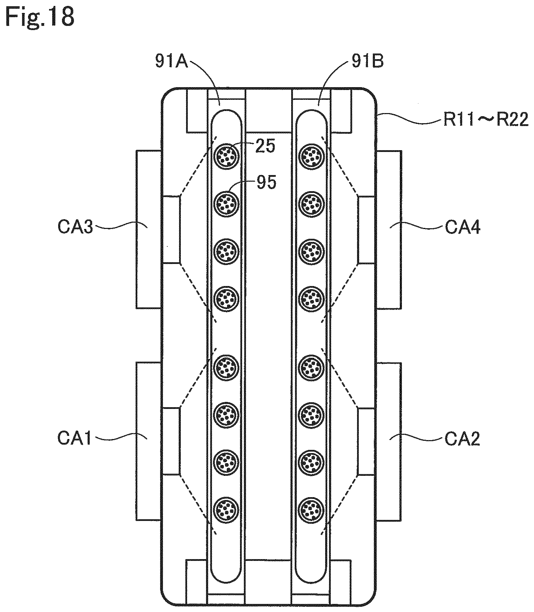

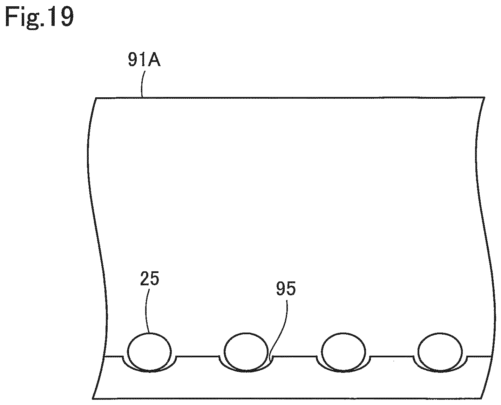

The camera module 51 may be provided on the side surface of the culture chamber Rn. In this case, the wells of the tray are linearly arranged. FIG. 18 illustrates a relationship between trays 91A and 91B and the culture chamber Rn in a case where the camera module 51 is provided on the side surface. As illustrated in FIG. 18, the eight wells 95 are linearly disposed in each of the trays 91A and 91B. Every four treatment eggs 25 in the eight wells 95 disposed in a row in the tray 91A are imaged by the two camera units CA1 and CA3 of a camera module 51A provided on the left side surface of the culture chamber Rn. The rest of the two camera units CA2 and CA4 of the camera module 51A are provided on the right side surface of the culture chamber Rn, and every four of the eight treatment eggs of the tray 91B are imaged. FIG. 19 schematically illustrates a state where this tray 91A is seen from the side of the camera unit CA1. The wells 95 are provided on the bottom surface of the tray 91A, and the treatment eggs 25 are accommodated therein. Consequently, a positional relationship between the camera units CA1 to CA4 and the treatment eggs 25 becomes stable, so that it is possible to easily capture images of the treatment eggs 25.

In this example, it is possible to reduce the widths of the trays 91A and 91B disposed in the one culture chamber Rn, so that it is possible to dispose the multiple culture chambers Rn and trays 91A and 91B in the one embryo culture device 10. Although, when the widths are narrowed, the trays 91A and 91B and the camera units are disposed close to each other, it is possible to clearly capture an image by using wide angle lenses even when the trays 91A and 91B are placed close to each other. When the wide angle lenses are used, distortion of an image becomes greater toward the periphery, and therefore it is necessary to greatly correct the image closer to the periphery in proportion to the distortion. Note that the camera module 51 may include camera units on front and rear side surfaces instead of the left and right side surfaces or in addition to the left and right side surfaces. The camera units may be provided on one of the left, right, front, and rear side surfaces. Alternatively, the camera units may be provided on the upper surface and the bottom surface to image treatment eggs from a plurality of directions. The images captured from a plurality of directions may be used for a purpose of making a determination using images captured from other directions in a case where some trouble occurs in an image captured from one direction such as a case where a captured image is not acquired due to breakdown of a camera. Furthermore, images captured from the plurality of directions may be used at a time of determination to enhance accuracy of determination.

When one treatment egg is imaged, a focus position is shifted, and a plurality of images are captured in the above embodiment. However, only one image of the one treatment egg may be captured at the predetermined interval without shifting the focus position in particular. Note that imaging may be performed using not only visible light, but also a range of near infrared, far infrared, ultraviolet light, or the like. Alternatively, a filter that allows only a predetermined wavelength range of visible light to transmit through may be provided, and imaging may be performed using only light that transmits through the filter.

When the wells 95 are illuminated, the wells 95 are illuminated from sides of the camera units CA1 to CA4 in the above embodiment. However, the embodiment is not limited to this configuration, and the wells 95 may be illuminated from the side or from below. In the embodiment, the light emitting elements LE1 to LE4 that are the light sources are provided in the LED module 83 that is a place apart from the camera units CA1 to CA4 to guide light to the vicinity of the camera units CA1 to CA4 through the light guide paths LG1 to LG4. However, the light emitting elements may be provided near the camera units CA1 to CA4. Alternatively, by contrast with this, the light sources may be provided at any place of the embryo culture device 10 instead of the time lapse unit 50, and light may be guided to the time lapse unit 50 through an optical fiber or the like.

The display module 52 that is the display unit is provided per time lapse unit 50 in the above embodiment. However, the display module 52 may be provided separately from the time lapse unit 50. One display unit may be provided to the plurality of time lapse units 50. Hence, a single display unit may be provided to the embryo culture device 10, or a plurality of display units associated with the plurality of time lapse units 50 may be provided. Furthermore, in this case, the display module 52 may be integrated with a form such as a large liquid crystal panel. Alternatively, a display of another computer or the like may be used to display using wireless LAN or wired LAN. Furthermore, an application may be installed in a mobile device such as a mobile telephone used by the embryologist to as a display. By so doing, even when the embryologist is not in a room in which the embryo culture device 10 is placed, the embryologist can check a situation of a treatment egg in real time. Alternatively, a patient who has provided a treatment egg and is undergoing, for example, fertility treatment can check a situation of the treatment egg. Furthermore, by so doing, two or more of a plurality of embryologists, or an embryologist and a patient can simultaneously check the situations of treatment eggs. Consequently, the embryologists, or the embryologist and the patient do not miss a change related to growth of treatment eggs such as a timing of fertilization, or determination of the growth.

The imaging timing (the start time or the interval) can be set per time lapse unit 50 provided per culture chamber Rn in the above embodiment. However, imaging start may be limited to a predetermined timing such as on the hour. Furthermore, the interval or the like may be also common between the plurality of time lapse units 50. Furthermore, a button displayed on the display module 52 is tapped to, for example, instruct imaging start. However, the instruction may be made by voice recognition together with other instructions. A dedicated instruction board may be prepared to operate a switch, a hardware button, or the like arranged on the instruction board.

In the above embodiment, the time lapse unit 50 also functions as the lid of the culture chamber Rn, is attached to a rim of the culture chamber Rn by an unillustrated hinge, and rotates to open and close the culture chamber Rn as illustrated in FIG. 2 to make it possible to attach and detach the tray 91. The time lapse unit 50 may be attached to the culture chamber Rn by a method other than rotation and movement. For example, the time lapse unit 50 may be simply placed on the upper surface of the culture chamber Rn. Alternatively, the time lapse unit 50 may be translated upward and downward. The time lapse unit 50 can be also configured to slide leftward and rightward or forward and backward.

The time lapse unit 50 is designed and attached as part of the embryo culture device 10 from the beginning in the above embodiment. However, the time lapse unit 50 may be exchanged with a lid of a culture chamber of an embryo culture device that has already been sold and installed. By so doing, the embryo culture device that has already been installed can be easily remade as an embryo culture device of a time lapse type.

Whether or not fertilization has normally occurred and progress situations of cleavage have been determined in the above embodiment. However, one of whether or not fertilization has normally occurred and the progress situations of cleavage may be determined. Furthermore, in addition to this determination, determination of whether or not a timing to take out a treatment egg from the culture has come, and determination of whether or not to exchange a culture medium with the treatment egg immersed in the well of the culture unit may be performed. The determination of whether or not a timing to take out a treatment egg from the culture has come may be learned by preparing for a treatment egg that has reached a predetermined stage a label associated with an image of a treatment egg to be taken out from the culture unit when the learned model 101 is generated. The determination of whether or not to exchange a culture medium with a treatment egg immersed in the well of the culture unit may be learned by adding to an image of the well a condition that, for example, an image of a culture medium captured together with a treatment egg gets dirty. The dirt or debris of the image of the culture medium can be easily learned from a decrease in luminance of the image of the culture medium or whether noise components corresponding to fine substances are great or little.

In a case where determination to take out the treatment egg or determination to exchange the culture medium is made, the camera module 51 and the display module 52 that function as a lid may be openable. Making the camera module 51 and the display module 52 openable may mean making the lid open immediately when the switches SW11 to SW22 are pushed, or making an actuator automatically open the lid when determination is made. The former generally assumes a case where the lid is made unopenable even when the switches SW11 to SW22 are touched by mistake during culturing.

The various embodiments of the present disclosure have been described above. However, the present disclosure is not limited to these embodiments, and can be naturally carried out as various embodiments without departing from the gist of the present disclosure. For example, the culture chamber Rn and the time lapse unit 50 may be paired as a minimum unit, these minimum units may be couplable in a left/right direction and/or a forward/backward direction, and a random number of culture chambers and a random number of time lapse units may be combined to form an embryo culture device. In the embryo culture device according to the present disclosure, the imaging unit is provided in association with each one of the trays held in the culture unit, so that, by coupling the imaging units and the trays in this way, it is easy to additionally install the culture units.

### C. Other Embodiment:

(1) The present disclosure includes carrying out an embryo culture device. This embryo culture device includes: a culture unit that accommodates a plurality of recess parts that can each accommodate a treatment egg, and keeps the recess parts in the culture environment; an imaging unit that continuously or intermittently captures an image including the treatment egg accommodated in the recess part without relatively moving the recess part; a storage unit that stores per treatment egg an egg image that is an image including the imaged treatment egg; a determination unit that applies a learned model generated in advance by supervised machine learning to the egg image of each treatment egg, and makes determination of growth of the treatment egg; and a display unit that reads from the storage unit an egg image of a treatment egg having been determined to satisfy a condition set in advance according to a result of the determination to display. This embryo culture device can make a determination of growth of treatment eggs using images obtained by imaging the treatment eggs accommodated and cultured in the recess parts without relatively moving the treatment eggs with respect to the imaging units, so that it is possible to reduce fluctuation of environment that is disadvantageous for the treatment eggs that are being cultured, and, moreover, accurately grasp situations of the treatment eggs that are being cultured and take a necessary measure.
   According to this embryo culture device, one imaging unit may be provided to the recess part, or one imaging unit may be provided to the plurality of recess parts. Alternatively, one imaging unit may be provided to a tray including a plurality of recess parts, or one imaging unit may be provided to a plurality of trays each including one or a plurality of recess parts. Either way, it is sufficient that the recess parts can be imaged without relatively moving the recess parts with respect to the imaging units. Note that the plurality of imaging units may be configured to image one recess part in an overlapping manner and cover an imaging mistake, or image one recess part from different angles using the plurality of imaging units to enhance accuracy of determination of growth. Furthermore, imaging may be performed intermittently or continuously. Intermittently means capturing images at a predetermined interval. The interval does not need to be the same time interval, and imaging may be performed at a shorter interval in a period during which a treatment egg is assumed to quickly change than in a period during which a treatment egg is not assumed to quickly change. Furthermore, continuously means capturing images as a movie. Continuous image capturing may be intermittently performed at the predetermined interval.
(2) According to this configuration, the determination unit may make the determination on an egg image that is not yet stored in the storage unit or the egg image that is read from the storage unit. By making a determination on the image that is not yet stored, a case will not happen where determination cannot be made due to a failure that may occur when the image is stored in or read from the storage unit. Furthermore, determination is not delayed by a time required for storage. On the other hand, by reading and making a determination on the stored image, it is possible to read and make a determination over and over when some failure occurs during determination.
(3) According to this configuration, the culture unit can accommodate a tray that includes the plurality of recess parts or can accommodate a plurality of trays that include the recess parts, the imaging unit may collectively image the plurality of recess parts, and the embryo culture device may further include a correction unit that divides per recess part a captured image obtained by collectively imaging the plurality of recess parts, and corrects as the egg image the image of the treatment egg present in the recess part from the divided image. By so doing, the imaging unit provided per tray can image the plurality of recess parts that can accommodate the treatment eggs at once, and subsequently divide the image per recess part and correct each divided image. Consequently, the image of the treatment egg accommodated in each recess part is corrected while imaging the plurality of recess parts at once, so that it is possible to more accurately make a determination of growth.
(4) According to this configuration, the correction may be a correction of matching a positional relationship between the plurality of recess parts and the imaging unit, or correction matching an imaging condition including at least a difference in brightness at a time of the imaging by the imaging unit. By so doing, it is possible to correct distortion aberration occurring in the image of the recess parts due to the positional relationship with the imaging units. Furthermore, by making correction matching an imaging condition that differs per tray such as a difference in brightness of a light or the like, it is possible to enhance accuracy of determination of growth.
(5) According to this configuration, the determination of growth of the treatment eggs by the learned model may be at least one of (A) determination of whether the treatment egg is fertilized or is not fertilized, (B) determination of whether cleavage of the treatment egg is normal or is not normal, (C) determination of whether a blastocyst is formed or is not formed in the treatment egg, (D) determination of whether or not a timing to take out the treatment egg from the culture unit has come, and (E) determination of whether or not to exchange a culture medium with the treatment egg immersed in the recess part of the culture unit. By so doing, it is possible to make various determinations by machine learning, and a person who performs culturing can perform more accurately set a timing of culturing or various processing such as processing of fertilized egg cryopreservation or embryo transfer subsequent to culturing.
(6) According to this configuration, the display unit may include an acceptance unit that displays the result of the determination and the egg image used for the determination, and then accepts applicability decision of an embryologist on the result of the determination. By so doing, by taking into account, for example, experiences of people such as embryologists who culture embryos instead of leaving determination to determination that uses a learned model generated by machine learning, it is possible to enhance accuracy of determination related to growth of treatment eggs. Note that it is not necessary to accept applicability decision of the embryologist for all determinations, and, for example, applicability decision of the embryologist on at least one of (A) determination of whether the treatment egg is fertilized or is not fertilized, (B) determination of whether cleavage of the treatment egg is normal or is not normal, and (C) determination of whether a blastocyst is formed or is not formed in the treatment egg may be accepted.
(7) This configuration may include a relearning unit that, when the applicability decision of the embryologist accepted by the acceptance unit denies the result of the determination displayed on the display unit, relearns and updates the learned model. By so doing, it is possible to update the learned model together with use of the embryo culture device, and further enhance accuracy of this determination.
(8) According to this configuration, the determination may be one determination of the (D) and the (E), and the display unit may display the result of the determination and the egg image used for the determination, and then make a lid provided to the culture unit openable. By so doing, it is not necessary to take out a treatment egg, and perform another processing of making it possible to open and close the lids one by one to exchange the culture media, so that it is possible to quickly perform work.
(9) The present disclosure includes carrying out a method for displaying a treatment egg kept in a culture environment and having been subjected to insemination treatment. This method includes: accommodating a plurality of recess parts that can each accommodate a treatment egg, and keeping the recess parts in the culture environment; providing an imaging unit at a predetermined position with respect to the recess part; continuously or intermittently capturing an image including the treatment egg accommodated in the recess part using the imaging unit; storing in a storage unit per treatment egg an egg image that is an image including the imaged treatment egg; applying a learned model generated in advance by supervised machine learning to the egg image of the treatment egg, and making a determination of growth of the treatment egg; and reading from the storage unit an egg image of a treatment egg having been determined to satisfy a condition set in advance according to a result of the determination to display on a display unit. By so doing, it is possible to display an image based on which determination of growth of treatment eggs has been made using images obtained by the imaging units prepared at predetermined positions by imaging the treatment eggs accommodated and cultured in the recess parts, so that it is possible to reduce fluctuation of environment that is disadvantageous for the treatment eggs that are being cultured, and, moreover, accurately grasp situations of the treatment eggs that are being cultured and provide necessary display.

The present disclosure is not limited to the above-described embodiments, and can be achieved by various configurations without departing from the gist of the present disclosure. For example, technical features in the embodiments corresponding to the technical features in each embodiment described in the Summary can be replaced or combined as appropriate to solve part or all of the above-described problems or achieve part or all of the above-described effects. Furthermore, unless these technical features are described as indispensable in this description, the technical features can be deleted as appropriate. For example, part of components achieved as hardware in the above embodiments can be achieved by software.

### Industrial Applicability

The embryo culture device, and the method for displaying treatment eggs kept in a culture environment and having been subjected to insemination treatment are available as a device that cultures the treatment eggs having been subjected to the insemination treatment and determine situations of the treatment eggs.

### Reference Signs List

CA1 ... CAMERA UNIT, H11 to H22 ... PANEL HEATER, L1, L2 ... LENS, LE1 to LE4 ... LIGHT EMITTING ELEMENT, LG1 to LG4 ... LIGHT GUIDE PATH, R11 to R22 ... CULTURE CHAMBER, SW11 to SW22 ... SWITCH, 9 ... CASE MAIN BODY, 10, 10A ... EMBRYO CULTURE DEVICE, 11, to 22 ... CULTURE UNIT, 25 ... TREATMENT EGG, 31 ... GAS PORT, 34 ... FILTER, 35, 36 ... FILTER PORT, 40 ... TRAY ACCOMMODATION PART, 42 ... EXPANDED PART, 43 ... SUPPLY PORT, 44 ... EXHAUST PORT, 50 ... TIME LAPSE UNIT, 51, 51A ... CAMERA MODULE, 52 ... DISPLAY MODULE, 53 ... CPU, 54 ... ROM, 55 ... RAM, 58 ... MEMORY INTERFACE, 59 ... MEMORY CARD, 60, 60A ... CONTROL UNIT, 61 ... CPU, 62 ... ROM, 63 ... RAM, 64 ... MEMORY INTERFACE, 65 ... MEMORY CARD, 66 ... GENERAL-PURPOSE I/O INTERFACE, 67 ... CULTURE CHAMBER INTERFACE, 71 ... DRIVING DEVICE, 72 ... WARNING DEVICE, 73 ... GASEOUS MIXTURE ADJUSTMENT DEVICE, 76 ... "START" BUTTON, 77a, 77b ... MESSAGE, 78 ... VARIOUS DISPLAY, 79a ... CULTURING END BUTTON, 79b ... CULTURING CONTINUE BUTTON, 81 ... CAMERA INTERFACE, 82 ... DISPLAY INTERFACE, 83 ... LED MODULE, 84 ... GASEOUS MIXTURE SUPPLY PIPE, 91, 91A, 91B ... TRAY, 92 ... PROTRUSION PART, 93 ... MINERAL OIL, 95 ... WELL

## Claims

1. An embryo culture device (10, 10A) that keeps in a culture environment (93) a treatment egg (25) having been subjected to insemination treatment, the embryo culture device (10, 10A) comprising:
a culture unit (11 to 22) that accommodates a plurality of recess parts (95) that can each accommodate a treatment egg (25), and keeps the recess parts (95) in the culture environment (93);
an imaging unit (CA1 to CA4) that continuously or intermittently captures an image including the treatment egg (25) accommodated in the recess part (95) without relatively moving the recess part (95);
a storage unit (59) that stores per treatment egg (25) an egg image that is an image including the imaged treatment egg (25);
a determination unit (56) that applies a learned model (101) generated in advance by supervised machine learning to the egg image of each treatment egg (25);
a display unit (52) that reads from the storage unit (59) an egg image of a treatment egg (25) having been determined to satisfy a condition set in advance according to a result of the determination to display,
**characterized in that**
the determination unit (56) makes determination of growth of the treatment egg (25), wherein
the display unit (52) includes an acceptance unit (79a, 79b) that displays the result of the determination and the egg image used for the determination, and then accepts an applicability decision of an embryologist on the result of the determination
the embryo culture device (10, 10A) further comprising a relearning unit (56) that, when the applicability decision of the embryologist accepted by the acceptance unit (79a, 79b) denies the result of the determination displayed on the display unit (52), relearns and updates the learned model (101).

2. The embryo culture device (10, 10A) according to claim 1, wherein the determination unit (56) makes the determination on an egg image that is not yet stored in the storage unit (59) or the egg image that is read from the storage unit (59).

3. The embryo culture device (10, 10A) according to claim 1 or 2, wherein
the culture unit (11 to 22) can accommodate a tray (91) that includes the plurality of recess parts (95) or can accommodate a plurality of trays that include the recess parts (95),
the imaging unit (CA1 to CA4) collectively images the plurality of recess parts (95), and
the embryo culture device (10, 10A) further comprises a correction unit that divides per recess part a captured image obtained by collectively imaging the plurality of recess parts (95), and corrects as the egg image the image of the treatment egg (25) existing in the recess part (95) from the divided image.

4. The embryo culture device (10, 10A) according to claim 3, wherein the correction is correction matching a positional relationship between the plurality of recess parts (95) and the imaging unit (CA1 to CA4), or correction matching an imaging condition including at least a difference in brightness at a time of the imaging by the imaging unit (CA1 to CA4).

5. The embryo culture device (10, 10A) according to any one of claims 1 to 4, wherein the determination of growth of the treatment egg (25) by the learned model (101) is at least one of
(B) determination of whether cleavage of the treatment egg (25) is normal or is not normal,
(C) determination of whether a blastocyst is formed or is not formed in the treatment egg (25), and
(D) determination of whether or not a timing to take out the treatment egg (25) from the culture unit (11 to 22) has come.

6. The embryo culture device (10, 10A) according to any one of claims 1 to 4, wherein the determination of growth of the treatment egg by the learned model is at least one of
(A) determination of whether the treatment egg (25) is fertilized or is not fertilized, and
(E) determination of whether or not to exchange a culture medium with the treatment egg (25) immersed in the recess part (95) of the culture unit (11 to 22).

7. The embryo culture device (10, 10A) according to claim 5 or 6, wherein
the determination is one determination of the (D) and the (E), and
the display unit (52) displays the result of the determination and the egg image used for the determination, and then makes a lid provided to the culture unit (11 to 22) openable.

8. A method for displaying a treatment egg (25) kept in a culture environment (93) and having been subjected to insemination treatment, the method comprising:
accommodating a plurality of recess parts (95) that can each accommodate a treatment egg (25), and keeping the recess parts (95) in the culture environment (93);
providing an imaging unit (CA1 to CA4) at a predetermined position with respect to the recess part;
continuously or intermittently capturing an image including the treatment egg (25) accommodated in the recess part using the imaging unit (CA1 to CA4);
storing in a storage unit (59) per treatment egg (25) an egg image that is an image including the imaged treatment egg (25);
applying a learned model (101) generated in advance by supervised machine learning to the egg image of the treatment egg (25), and making a determination of growth of the treatment egg (25); and
reading from the storage unit (59) an egg image of a treatment egg (25) having been determined to satisfy a condition set in advance according to a result of the determination to display on a display unit (52),
**characterized by** further comprising
when applying a learned model (101), making a determination of growth of the treatment egg (25),
displaying the result of the determination and the egg image used for the determination, and then accepting an applicability decision of an embryologist on the result of the determination, and
when the applicability decision of the embryologist accepted by the acceptance unit (79a, 79b) denies the result of the determination displayed on the display unit, relearning and updating the learned model (101).

## Patentansprüche

1. Embryonenkultivierungsvorrichtung (10, 10A), die ein einer Befruchtungsbehandlung unterzogenes behandeltes Ei (25) in einer Kultivierungsumgebung (93) hält, wobei die Embryonenkultivierungsvorrichtung (10, 10A) aufweist:
eine Kultivierungseinheit (11 bis 22), die mehrere Aussparungen (95) aufnimmt, von denen jede ein behandeltes Ei (25) aufnehmen kann, und die die Aussparungen (95) in der Kultivierungsumgebung (93) hält;
eine Abbildungseinheit (CA1 bis CA4), die kontinuierlich oder intermittierend ein Bild aufnimmt, das das in der Aussparung (95) untergebrachte behandelte Ei (25) enthält, ohne die Aussparung (95) relativ zu bewegen;
eine Speichereinheit (59), die pro behandeltem Ei (25) ein Ei-Bild speichert, bei dem es sich um ein Bild handelt, das das abgebildete behandelte Ei (25) enthält;
eine Bestimmungseinheit (56), die ein zuvor durch überwachtes maschinelles Lernen generiertes, trainiertes Modell (101) auf das Eibild jedes behandelten Eis (25) anwendet;
eine Anzeigeeinheit (52), die aus der Speichereinheit (59) ein Eibild eines behandelten Eis (25) ausliest, bei dem gemäß einem Ergebnis der Bestimmung festgestellt wurde, dass es eine vorab festgelegte Bedingung erfüllt, um es anzuzeigen,
**dadurch gekennzeichnet, dass**
die Bestimmungseinheit (56) eine Bestimmung des Wachstums des behandelten Eis (25) vornimmt, wobei
die Anzeigeeinheit (52) eine Akzeptanzeinheit (79a, 79b) aufweist, die das Ergebnis der Bestimmung und das für die Bestimmung verwendete Eibild anzeigt und anschließend eine Anwendbarkeitsentscheidung eines Embryologen bezüglich des Ergebnisses der Bestimmung akzeptiert,
die Embryonenkultivierungsvorrichtung (10, 10A) ferner eine Nachlern-Einheit (56) aufweist, die, wenn die von der Akzeptanzeinheit (79a, 79b) akzeptierte Anwendbarkeitsentscheidung des Embryologen das auf der Anzeigeeinheit (52) angezeigte Ergebnis der Bestimmung ablehnt, das trainierte Modell (101) neu trainiert und aktualisiert.

2. Embryonenkultivierungsvorrichtung (10, 10A) nach Anspruch 1, wobei die Bestimmungseinheit (56) die Bestimmung anhand eines Eibildes vornimmt, das noch nicht in der Speichereinheit (59) gespeichert ist, oder anhand des Eibildes, das aus der Speichereinheit (59) ausgelesen wird.

3. Embryonenkultivierungsvorrichtung (10, 10A) nach Anspruch 1 oder 2, wobei
die Kultivierungseinheit (11 bis 22) eine Schale (91) aufnehmen kann, die die mehreren Aussparungen (95) aufweist, oder mehrere Schalen aufnehmen kann, die die Aussparungen (95) umfassen,
die Abbildungseinheit (CA1 bis CA4) die mehreren Aussparungen (95) gemeinsam abbildet, und
die Embryonenkultivierungsvorrichtung (10, 10A) ferner eine Korrektureinheit aufweist, die ein durch die gemeinsame Abbildung der mehreren Aussparungen (95) erhaltenes Bild pro Aussparung aufteilt und aus dem aufgeteilten Bild das Bild des in der Aussparung (95) befindlichen behandelten Eis (25) als Eibild korrigiert.

4. Embryonenkultivierungsvorrichtung (10, 10A) nach Anspruch 3, wobei die Korrektur eine Korrektur ist, die eine Positionsbeziehung zwischen den mehreren Aussparungen (95) und der Abbildungseinheit (CA1 bis CA4) angleicht, oder eine Korrektur, die eine Abbildungsbedingung angleicht, die zumindest einen Helligkeitsunterschied zum Zeitpunkt der Abbildung durch die Abbildungseinheit (CA1 bis CA4) aufweist.

5. Embryonenkultivierungsvorrichtung (10, 10A) nach einem der Ansprüche 1 bis 4, wobei die Bestimmung des Wachstums des behandelten Eis (25) durch das trainierte Modell (101) mindestens eines der folgenden ist:
(B) die Bestimmung, ob die Zellteilung des zu behandelnden Eis (25) normal ist oder nicht,
(C) die Bestimmung, ob sich in dem zu behandelnden Ei (25) eine Blastozyste bildet oder nicht, und
(D) die Bestimmung, ob der Zeitpunkt für die Entnahme des zu behandelnden Eis (25) aus der Kultivierungseinheit (11 bis 22) gekommen ist oder nicht.

6. Embryonenkultivierungsvorrichtung (10, 10A) nach einem der Ansprüche 1 bis 4, wobei die Bestimmung des Wachstums des zu behandelnden Eis durch das trainierte Modell (101) mindestens eine der folgenden ist:
(A) die Bestimmung, ob das zu behandelnde Ei (25) befruchtet ist oder nicht, und
(E) die Bestimmung, ob das Kulturmedium, in das das zu behandelnde Ei (25) in der Aussparung (95) der Kultivierungseinheit (11 bis 22) eingetaucht ist, ausgetauscht werden muss oder nicht.

7. Embryonenkultivierungsvorrichtung (10, 10A) nach Anspruch 5 oder 6, wobei
die Bestimmung eine der Bestimmungen (D) und (E) ist, und
die Anzeigeeinheit (52) das Ergebnis der Bestimmung und das für die Bestimmung verwendete Eibild anzeigt und anschließend einen an der Kultivierungseinheit (11 bis 22) vorgesehenen Deckel öffenbar macht.

8. Verfahren zum Anzeigen eines in einer Kultivierungsumgebung (93) aufbewahrten und einer Befruchtungsbehandlung unterzogenen behandelten Eis (25), wobei das Verfahren aufweist:
Bereitstellen einer Vielzahl von Aussparungen (95), von denen jede ein behandeltes Ei (25) aufnehmen kann, und Aufbewahren der Aussparungen (95) in der Kultivierungsumgebung (93);
Bereitstellen einer Abbildungseinheit (CA1 bis CA4) an einer vorbestimmten Position in Bezug auf die Aussparung;
kontinuierliches oder intermittierendes Aufnehmen eines Bildes, das das in dem Aufnahmeteil untergebrachte behandelte Ei (25) enthält, unter Verwendung der Abbildungseinheit (CA1 bis CA4);
Speichern eines Eibildes, bei dem es sich um ein Bild handelt, das das abgebildete behandelte Ei (25) enthält, in einer Speichereinheit (59) für jedes behandelte Ei (25);
Anwenden eines zuvor durch überwachtes maschinelles Lernen generierten trainierten Modells (101) auf das Eibild des behandelten Eis (25) und Vornehmen einer Bestimmung des Wachstums des behandelten Eis (25); und
Auslesen eines Eibildes eines behandelten Eis (25) aus der Speichereinheit (59), bei dem gemäß einem Ergebnis der Bestimmung festgestellt wurde, dass es eine vorab festgelegte Bedingung erfüllt, um es auf einer Anzeigeeinheit (52) anzuzeigen,
**dadurch gekennzeichnet, dass** es weiter aufweist
Vornehmen einer Bestimmung des Wachstums des behandelten Eis (25) bei der Anwendung eines trainierten Modells (101),
Anzeigen des Ergebnisses der Bestimmung und des für die Bestimmung verwendeten Eibildes sowie anschließendes Akzeptieren einer Anwendbarkeitsentscheidung eines Embryologen bezüglich des Ergebnisses der Bestimmung, und
wenn die von der Akzeptanzeinheit (79a, 79b) akzeptierte Anwendbarkeitsentscheidung des Embryologen das auf der Anzeigeeinheit angezeigte Ergebnis der Bestimmung ablehnt, erneutes Trainieren und Aktualisieren des trainierten Modells (101).

## Revendications

1. Un dispositif de culture d'embryons (10, 10A) destiné à maintenir, dans un environnement de culture (93), un œuf de traitement (25) ayant subi un traitement d'insémination, le dispositif de culture d'embryons (10, 10A) comprenant :
une unité de culture (11 à 22) qui comporte une pluralité de cavités (95) pouvant chacune recevoir un œuf de traitement (25), et qui maintient les cavités (95) dans l'environnement de culture (93) ;
une unité d'imagerie (CA1 à CA4) qui capture de manière continue ou intermittente une image comprenant l'œuf de traitement (25) logé dans la cavité (95) sans déplacer celle-ci par rapport à l'unité d'imagerie ;
une unité de stockage (59) qui stocke, pour chaque œuf de traitement (25), une image d'œuf qui est une image comprenant l'œuf de traitement (25) capturé ;
une unité de détermination (56) qui applique un modèle appris (101), généré au préalable par apprentissage automatique supervisé, à l'image de chaque œuf de traitement (25) ;
une unité d'affichage (52) qui lit dans l'unité de stockage (59) une image d'œuf d'un œuf de traitement (25) dont il a été déterminé qu'il satisfait à une condition définie au préalable, conformément au résultat de la détermination, afin de l'afficher,
**caractérisé en ce que**
l'unité de détermination (56) effectue une détermination de la croissance de l'œuf de traitement (25), dans lequel
l'unité d'affichage (52) comprend une unité d'acceptation (79a, 79b) qui affiche le résultat de la détermination et l'image de l'œuf utilisée pour celle-ci, puis accepte une décision d'applicabilité d'un embryologiste concernant le résultat de la détermination,
le dispositif de culture d'embryons (10, 10A) comprenant en outre une unité de réapprentissage (56) qui, lorsque la décision d'applicabilité de l'embryologiste acceptée par l'unité d'acceptation (79a, 79b) conteste le résultat de la détermination affiché sur l'unité d'affichage (52), réapprend et met à jour le modèle appris (101).

2. Le dispositif de culture d'embryons (10, 10A) selon la revendication 1, dans lequel l'unité de détermination (56) effectue la détermination sur une image d'œuf qui n'est pas encore stockée dans l'unité de stockage (59) ou sur l'image d'œuf lue à partir de l'unité de stockage (59).

3. Le dispositif de culture d'embryons (10, 10A) selon la revendication 1 ou 2, dans lequel
l'unité de culture (11 à 22) peut recevoir un plateau (91) comportant une pluralité de cavités (95) ou peut recevoir une pluralité de plateaux comportant les cavités (95),
l'unité d'imagerie (CA1 à CA4) capture collectivement l'image de la pluralité de cavités (95), et
le dispositif de culture d'embryons (10, 10A) comprend en outre une unité de correction qui divise, pour chaque partie en creux, une image capturée obtenue par l'imagerie collective de la pluralité de cavités (95), et qui corrige, en tant qu'image d'œuf, l'image de l'œuf de traitement (25) présent dans la cavité (95) à partir de l'image divisée.

4. Le dispositif de culture d'embryons (10, 10A) selon la revendication 3, dans lequel la correction consiste soit à ajuster la relation de position entre la pluralité de cavités (95) et l'unité d'imagerie (CA1 à CA4), soit à ajuster les conditions d'imagerie, notamment en tenant compte d'au moins une différence de luminosité au moment de l'imagerie par l'unité d'imagerie (CA1 à CA4).

5. Le dispositif de culture d'embryons (10, 10A) selon l'une quelconque des revendications 1 à 4, dans lequel la détermination de la croissance de l'œuf de traitement (25) par le modèle appris (101) consiste en au moins l'une des opérations suivantes :
(B) la détermination du caractère normal ou anormal de la segmentation de l'œuf de traitement (25),
(C) la détermination de la formation ou non d'un blastocyste dans l'œuf de traitement (25), et
(D) la détermination du moment opportun ou non pour retirer l'œuf de traitement (25) de l'unité de culture (11 à 22).

6. Le dispositif de culture d'embryons (10, 10A) selon l'une quelconque des revendications 1 à 4, dans lequel la détermination de la croissance de l'œuf de traitement par le modèle appris (101) consiste en au moins l'une des opérations suivantes :
(A) déterminer le fait que l'œuf de traitement (25) est fécondé ou non, et
(E) déterminer la nécessité ou non de remplacer le milieu de culture dans lequel l'œuf de traitement (25) est immergé dans la cavité (95) de l'unité de culture (11 à 22).

7. Le dispositif de culture d'embryons (10, 10A) selon la revendication 5 ou 6, dans lequel
la détermination consiste en une seule détermination des paramètres (D) et (E), et
l'unité d'affichage (52) affiche le résultat de la détermination ainsi que l'image de l'œuf utilisée pour cette détermination, puis permet d'ouvrir le couvercle prévu sur l'unité de culture (11 à 22).

8. Un procédé permettant de visualiser un œuf de traitement (25) conservé dans un environnement de culture (93) et ayant subi un traitement d'insémination, le procédé comprenant les étapes suivantes :
la mise en place d'une pluralité de cavités (95) pouvant chacune recevoir un œuf de traitement (25), et la conservation des cavités (95) dans l'environnement de culture (93) ;
mettre en place une unité d'imagerie (CA1 à CA4) à une position prédéterminée par rapport à l'alvéole ;
capturer, en continu ou par intermittence, une image comprenant l'œuf de traitement (25) logé dans la cavité à l'aide de l'unité d'imagerie (CA1 à CA4) ;
stocker, dans une unité de stockage (59), pour chaque œuf de traitement (25), une image d'œuf qui est une image comprenant l'œuf de traitement (25) capturé ;
appliquer un modèle appris (101), généré au préalable par apprentissage automatique supervisé, à l'image d'œuf de l'œuf de traitement (25), et la détermination de la croissance de l'œuf de traitement (25) ; et
lire dans l'unité de stockage (59) une image d'un œuf de traitement (25) pour lequel il a été déterminé qu'il satisfait à une condition définie au préalable, conformément au résultat de la détermination, afin de l'afficher sur une unité d'affichage (52),
**caractérisé en ce qu'**il comprend en outre
lors de l'application d'un modèle appris (101), effectuer une détermination de la croissance de l'œuf de traitement (25),
afficher le résultat de cette détermination et de l'image de l'œuf utilisée pour celle-ci, puis accepter une décision d'applicabilité émise par un embryologiste concernant le résultat de la détermination, et
lorsque la décision d'applicabilité de l'embryologiste acceptée par l'unité d'acceptation (79a, 79b) conteste le résultat de la détermination affiché sur l'unité d'affichage, le réapprentissage et la mise à jour du modèle appris (101).
